# EUROPEAN PATENT SPECIFICATION

(11) **EP 3 082 588 B1**
(45) Date of publication and mention of the grant of the patent: **19.09.2018**
(21) Application number: 15705739.9
(22) Date of filing: 28.01.2015
(51) Int. Cl.: A61B 5/042, A61B 5/00, A61B 18/14, A61N 1/05, A61B 90/00, A61B 5/053, A61B 5/06, A61B 18/12

(54) **ELONGATE MEDICAL DEVICES INCORPORATING A FLEXIBLE SUBSTRATE, A SENSOR, AND ELECTRICALLY-CONDUCTIVE TRACES**
LÄNGLICHE MEDIZINPRODUKTE MIT EINEM FLEXIBLEN SUBSTRAT, SENSOR UND ELEKTRISCH LEITFÄHIGEN SPUREN
DISPOSITIFS MÉDICAUX ALLONGÉS INCORPORANT UN SUBSTRAT FLEXIBLE, UN CAPTEUR, ET DES PISTES ÉLECTRIQUEMENT CONDUCTRICES

(30) Priority: 28.01.2014 US 201461932499 P; 28.01.2014 US 201461932386 P; 18.09.2014 US 201462052255 P
(43) Date of publication of application: 26.10.2016
(73) Proprietor: St. Jude Medical International Holding S.à r.l., 1417 Luxembourg (LU)
(72) Inventor: STERRETT, Terry, Huntington Beach, California 92646 (US); JENSRUD, Allyn, Burnsville, Minnesota 55306 (US)
(74) Representative: Kramer Barske Schmidtchen Patentanwälte PartG mbB
(86) International application number: PCT/US2015/013301
(87) International publication number: WO 2015/116687

(56) References cited:
- EP-A1- 2 032 201
- EP-A2- 0 989 384
- US-A1- 2005 085 716
- US-A1- 2005 283 067
- US-A1- 2013 066 194
- US-A1- 2013 169 272

## Description

### BACKGROUND

### a. Technical Field

The instant disclosure relates to elongate medical devices, including the elongate medical devices that include a flexible substrate, a sensor, and electrically-conductive traces.

### b. Background Art

A wide variety of elongate medical devices are inserted into the body to diagnose and treat various medical conditions. Catheters, for example, are used to perform a variety of tasks within human bodies and other bodies including the delivery of medicine and fluids, the removal of bodily fluids and the transport of surgical tools and instruments. In the diagnosis and treatment of atrial fibrillation, for example, catheters may be used to deliver electrodes to the heart for electrophysiological mapping of the surface of the heart and to deliver ablative energy to the surface among other tasks. Catheters may be inserted into the patient with an introducer through which the catheter is inserted, for example, and/or may be guided to a target site through, in part, the use of a guidewire over which the catheter is inserted.

The position and orientation of elongate medical devices may be determined with, for example, an electric-field or magnetic-field based positioning system. Such positioning systems typically function in conjunction with one or more position sensors (e.g., electrodes and electromagnetic coil sensors) disposed on or in the elongate medical devices. The sensors are generally electrically connected to a conductor in an electrical cable in the medical device to transfer a detected signal to the positioning system for further processing for extracting information from that signal that is indicative of the position of the sensor. Such an electrical connection can be made my soldering the respective leads of the sensor and cable together at a solder joint. However, the leads and sensors themselves are delicate and, thus, can be damaged during fabrication and assembly of the medical device. Moreover, due to the small diameter (*e.g.,* 10µm) and fragility of the leads, the leads can break at or near the solder joint during or after testing (*e.g.,* stress rupture testing) of the medical device. Another mechanism for electrically coupling the sensor and cable is by coupling the respective leads of the sensor and cable to a rigid or flexible substrate disposed therebetween. In light of the relatively small dimensions that such sensors must exhibit in order to fit into a typical medical device, fabrication of such sensors can be complicated, may occupy undesirable amounts of radial space in the device, and/or may involve fabrication methods that are more costly than desired.

Medical devices including sensors and cables are known from EP 2 032 201 A1, EP 0 989 384 A2, US 2013/066194 A1, US 2013/169272 A1, US 2005/085716 A1 and US 2005/0283067 A1.

The foregoing discussion is intended only to illustrate the present field and should not be taken as a disavowal of claim scope.

### BRIEF SUMMARY

The invention is defined in the claims.

### BRIEF DESCRIPTION OF THE DRAWINGS

Figure 1 is a plan view of an exemplary elongate medical device.
Figure 2 is an isometric view of an exemplary embodiment of a distal end portion of an elongate medical device.
Figures 3A-9 are isometric views of an inner tube that may form a part of an elongate medical device shaft illustrating various stages in a first exemplary embodiment of a method of depositing electrically-conductive traces on the inner tube.
Figures 10A-14 are isometric views of an elongate medical device shaft assembly illustrating various stages in a method of assembling the elongate medical device shaft.
Figure 15 is a sectional view of a portion of a medical device for diagnosis or treatment of tissue in accordance with one embodiment of the present teachings (not forming part of the claimed invention).
Figure 16 is a perspective view of a partially formed electronic subassembly of the medical device of Figure 15 in accordance with one embodiment of the present teachings.
Figure 17 is a perspective view of the electronic subassembly of Figure 16 in a deformed state.
Figure 18 is a perspective view of the electronic subassembly of Figure 17 in an encapsulated state.
Figure 19 is a perspective view of a partially formed electronic subassembly of the medical device of Figure 15 in accordance with another embodiment of the present teachings (not forming part of the claimed invention).
Figure 20 is a perspective view of the electronic subassembly of Figure 19 in a deformed state.
Figure 21 is a perspective view of the electronic subassembly of Figure 20 in an encapsulated state.
Figure 22 is a flow chart diagram illustrating various embodiments of a method for fabricating a medical device for diagnosis or treatment of tissue in a body in accordance with the present teachings.
Figure 23A is a plan view of an exemplary embodiment of a stage of build-up of a guidewire assembly.
Figure 23B is an isometric view of a portion of the guidewire assembly of Figure 23A.
Figure 24A is a plan view of an exemplary embodiment of a stage of build-up of a guidewire assembly.
Figure 24B is an isometric view of a portion of the guidewire assembly of Figure 24A.
Figure 25A is a plan view of an exemplary embodiment of a stage of build-up of a guidewire assembly.
Figure 25B is an isometric view of a portion of the guidewire assembly of Figure 25A.
Figure 26 is an isometric view of a distal end portion of an exemplary embodiment of a stage of build-up of a guidewire assembly.
Figure 27 is an isometric view of an intermediate portion of an exemplary embodiment of a stage of build-up of a guidewire assembly.
Figure 28 is an isometric view of a proximal end portion of an exemplary embodiment of a stage of build-up of a guidewire assembly.
Figure 29 is an isometric view of an alternate embodiment of an intermediate portion of a stage of build-up of a guidewire assembly.
Figure 30 is an isometric view of an alternate embodiment of an intermediate portion of a stage of build-up of a guidewire assembly.
Figure 31 is a diagrammatic view of a guidewire prolapsed within a vessel of a body of a patient.
Figure 32 is a diagrammatic top view of an initial stage of manufacture of an exemplary embodiment of a substrate that may find use with the medical devices of this disclosure.
Figure 33A is a diagrammatic top view of a later stage of manufacture of the substrate embodiment of Figure 32.
Figure 33B is a diagrammatic side view of the substrate stage of manufacture of Figure 33A.

### DETAILED DESCRIPTION

Various embodiments are described herein to various apparatuses, systems, and/or methods. Numerous specific details are set forth to provide a thorough understanding of the overall structure, function, manufacture, and use of the embodiments as described in the specification and illustrated in the accompanying drawings. It will be understood by those skilled in the art, however, that the embodiments may be practiced without such specific details. In other instances, well-known operations, components, and elements have not been described in detail so as not to obscure the embodiments described in the specification. Those of ordinary skill in the art will understand that the embodiments described and illustrated herein are non-limiting examples, and thus it can be appreciated that the specific structural and functional details disclosed herein may be representative and do not necessarily limit the scope of the embodiments, the scope of which is defined solely by the appended claims.

Reference throughout the specification to "various embodiments," "some embodiments," "one embodiment," or "an embodiment", or the like, means that a particular feature, structure, or characteristic described in connection with the embodiment is included in at least one embodiment. Thus, appearances of the phrases "in various embodiments," "in some embodiments," "in one embodiment," or "in an embodiment", or the like, in places throughout the specification are not necessarily all referring to the same embodiment. Furthermore, the particular features, structures, or characteristics may be combined in any suitable manner in one or more embodiments. Thus, the particular features, structures, or characteristics illustrated or described in connection with one embodiment may be combined, in whole or in part, with the features structures, or characteristics of one or more other embodiments without limitation given that such combination is not illogical or non-functional.

It will be appreciated that the terms "proximal" and "distal" may be used throughout the specification with reference to a clinician manipulating one end of an instrument used to treat a patient. The term "proximal" refers to the portion of the instrument closest to the clinician and the term "distal" refers to the portion located furthest from the clinician. It will be further appreciated that for conciseness and clarity, spatial terms such as "vertical," "horizontal," "up," and "down" may be used herein with respect to the illustrated embodiments. However, surgical instruments may be used in many orientations and positions, and these terms are not intended to be limiting and absolute.

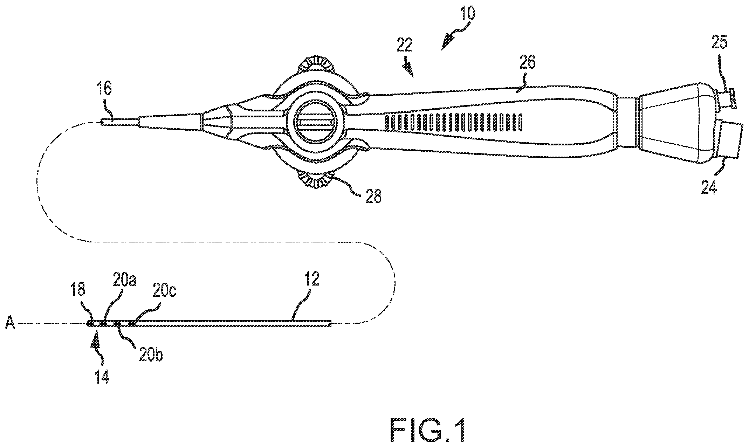

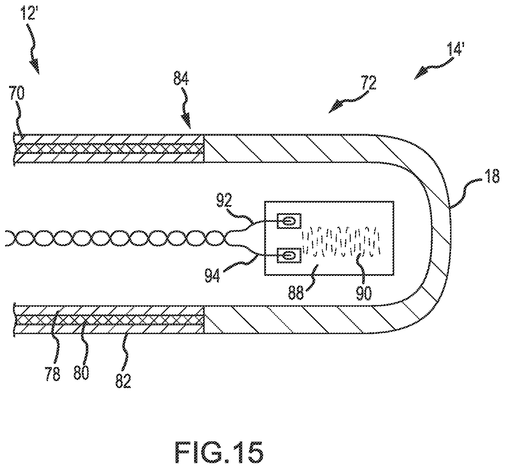

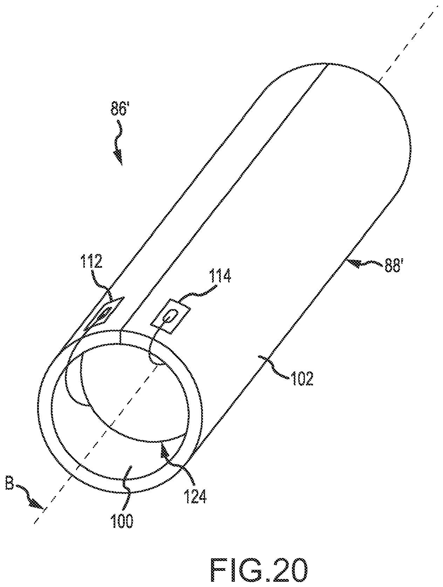

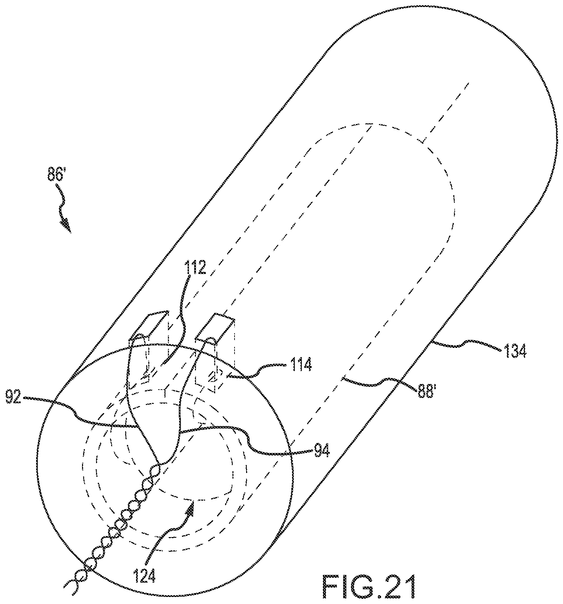

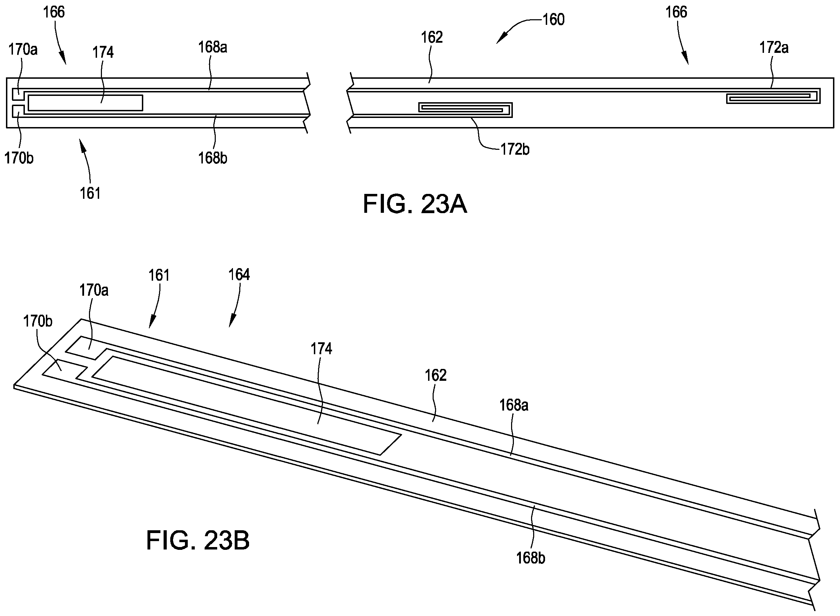

Before proceeding to a detailed description of embodiments, a brief description of the various aspects of this disclosure is first set forth. This disclosure generally includes elongate medical devices such as guidewires, catheters, and introducers that incorporate sensor assemblies that include a sensor, a flexible substrate, and electrically-conductive traces on the substrate. A first aspect of the disclosure, set forth below in conjunction with Figures 1-14, generally includes electrode sensors electrically coupled with electrically-conductive traces on a flexible inner tube. This first aspect is entitled "Elongate Medical Device Incorporating Electrically-Conductive Traces on An Inner Tube" below. A second aspect of this disclosure, set forth below with respect to Figures 15-22, generally includes a coil sensor (*e.g.,* for use with an electromagnetic positioning system) disposed within a rolled flexible substrate. This second aspect is entitled "Elongate Medical Device Incorporating Coil Sensor and Flexible Substrate" below. A third aspect of this disclosure, set forth below with respect to Figures 23A-33B, generally includes a coil sensor disposed within a rolled flexible substrate that may extend over substantially the entire length of the medical device. This third aspect is entitled "Elongate Medical Device Incorporating Coil Sensor and Full-Length Flexible Substrate" below.

Further differences between and common aspects among the three aspects of this disclosure will become clear in the description below. It should be noted that, although described separately, the features of the different aspects of the disclosure may be combined in various embodiments to arrive at embodiments different from those explicitly illustrated and/or described in this disclosure. Still further, it should be understood that materials, structures, coupling techniques, and the like described with respect to one aspect of this disclosure may also find use with other aspects of this disclosure. Finally, the claims are not limited to a single aspect of this disclosure except as explicitly recited in the claims.

*Elongate Medical Device Incorporating Electrically-Conductive Traces on An Inner Tube.* Referring now to the figures, in which like numerals indicate the same or similar elements in the various views, Figure 1 is a plan view of an exemplary elongate medical device 10. The elongate medical device 10 may be a catheter, introducer, or other elongate medical device type. The elongate medical device 10 will be referred to herein as a catheter for ease of description (*i.e.,* catheter 10). It should be understood, though, that the elongate medical device is not limited to a catheter.

The catheter 10 may include an elongate tubular shaft 12 defining a longitudinal axis A and having a distal end portion 14 and a proximal end portion 16, a tip electrode 18, a number of ring electrodes 20a, 20b, 20c (which may be referred to collectively as the ring electrodes 20 or individually as a ring electrode 20), and a handle 22 coupled with the catheter shaft 12. The handle 22 may include one or more electromechanical connectors 24 configured to allow the catheter 10, and the electrodes 18, 20 thereof, in particular, to be coupled with components or subsystems of, for example, an electrophysiology (EP) laboratory system. Such components or subsystems may comprise, for example and without limitation, a visualization, navigation, and/or mapping system, an EP monitoring and recording system (*e.g.,* for monitoring and/or recording electrocardiograms (EGM), cardiac signals, etc.), a tissue contact sensing system, an ablation system, a cardiac stimulation system (*i.e.,* EP stimulator), and the like. An exemplary system is shown in U.S. patent application publication no. 2012/0029504.

The catheter 10 may further comprise one or more fluid connectors 25 configured to provide the catheter 10, and particularly the shaft 12, with connectivity between one or more fluid lumen(s) in the shaft 12 and external systems. The fluid connector 25 may thus be fluidly coupled with one or more fluid lumens in the shaft 12 and/or handle 22 and may be configured for connection with a source or destination of such fluids such as, for example only, a gravity feed or pump for irrigation fluids.

In addition to and/or instead of one or more electrodes 18, 20, the catheter 10 may be equipped with one or more additional types of sensors. For example, the catheter 10 may be equipped with one or more coil sensors, temperature sensors, pressure sensors, and/or other sensors. Additionally, some or all of the steps, methods, and procedures described and/or illustrated herein related to the manufacturing, assembly, and use of electrodes 18, 20 on the catheter 10 may also apply to other types of sensors disposed on or in the catheter 10.

The handle 22 may be disposed at the proximal end portion 16 of the shaft 12. The handle 22 may provide a location for a clinician to hold the catheter 10 and may further provide means for steering or guiding the shaft 12 within the body of a patient.

The handle 22 may comprise a housing 26. The housing 26mmay be of a unitary construction or may be constructed of a plurality of pieces that are configured to be assembled together. In a multi-piece embodiment, the housing 26 may be coupled together in any number of ways known in the art, such as, for example, by press fit or interference coupling techniques, by complementary interlocking members, by conventional fasteners or adhesives, or any other techniques known in the art.

Within the housing 26, one or more wires may be provided to electrically couple the electromechanical connector 24 with the electrical infrastructure of the shaft 12. For example, in an embodiment, one wire may be provided for each electrical trace on a surface of the shaft, as shown and described in detail below. A wire in the housing 26 may be soldered to an electrical trace on one end, for example, and soldered or otherwise electrically coupled to the electromechanical connector 24 within the housing 26 on the other end.

In an exemplary embodiment, the catheter 10 may further comprise a deflection mechanism 28 associated with the handle 22 of the catheter 10. The deflection mechanism 28 may be coupled with a pull assembly (not shown) disposed at or in the distal end portion 14 of the shaft 12. The combination of the deflection mechanism 28 and the pull assembly provides a means by which a user or physician can effect movement (*e.g.,* deflection) of the distal end portion 14 in one or more directions, and therefore, allows the physician to steer the catheter shaft 12.

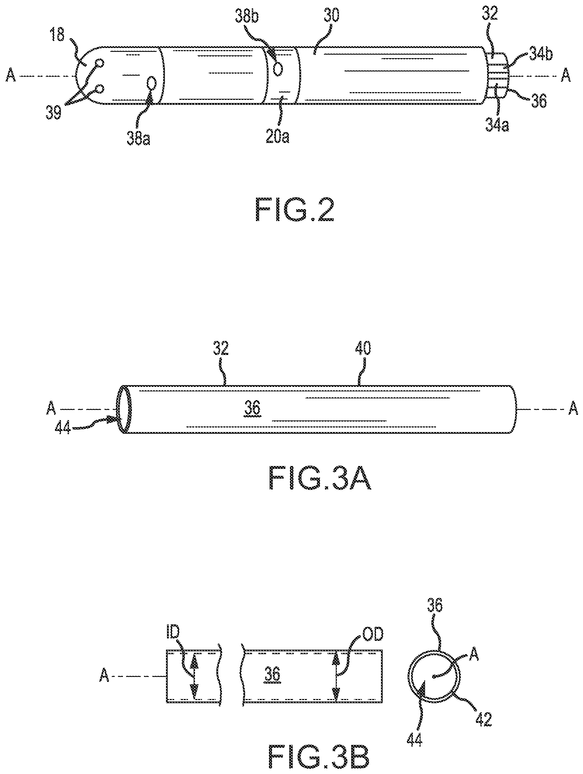

Figure 2 is an isometric view of an embodiment of the distal end portion 14 of the catheter 10, with a portion of an outer tube 30 of the shaft 12 cut away to expose an inner tube 32. The inner tube 32 may extend within the outer tube 30, and a first electrically-conductive trace 34a and a second electrically-conductive trace 34b may be disposed on an outer surface 36 of the inner tube 32. The distal end portion 14 may include, as noted above, a tip electrode 18 and one or more ring electrodes 20 (one such ring electrode 20a is shown in Figure 2). The tip electrode 18 may define a first bore 38a (*i.e.,* via), and the ring electrode may define a second bore 38b (*i.e.,* via). Bores 38a and 38b and other bores shown and/or described herein may be referred to collectively as the bores 38 or individually as the bore 38. Each bore 38 may extend, substantially orthogonal to the axis A of the shaft 12, from an exterior surface of the electrode 18, 20 to a portion of a respective one of the traces 34. Thus, the first bore 38a may extend from an exterior surface of the tip electrode 18, through a portion of the body of the electrode 18 to a portion of a first trace 34a, and the second bore 38b may extend from the exterior surface of the ring electrode 20a to a portion of a second trace 34b. Thus, the first bore 38a may be axially coincident with a portion of the first trace 34a, and the second bore 38b may be axially coincident with a portion of the second trace 34b.

The first bore 38a may be filled with an element (*e.g.,* a material) that electrically couples the tip electrode 18 with the first trace 34a, and the second bore 38b may also be filled with an element (*e.g.,* a material) that electrically couples the band electrode 20a with the second trace 34b. For example, in an embodiment, each bore 38 may be filled with an electrically-conductive adhesive. Such an electrically-conductive adhesive may include, for example only, silver-filled polyurethane, epoxy, and/or silicone adhesive.

The tip electrode 18 may further include one or more irrigation ports 39, in an embodiment. Irrigation fluid may be provided from a system disposed at the proximal end of the catheter (*e.g.,* a gravity feed or pump, as noted above) and may flow through the irrigation ports 39 in order to, for example only, cool the tip electrode. Additional details regarding irrigated electrodes may be found, for example, in U.S. Patent Nos. 8,517,999 and 8,187,267.

In an embodiment, the inner tube 32 may comprise some or all of a fluid lumen for the catheter 10. The fluid lumen may be configured to carry one or more fluids (*e.g.,* irrigation fluid) between the handle of the finished device and the distal tip of the finished device. Fluid may flow through the inner tube 32 to the irrigation ports 39, in an embodiment.

Referring to Figures 1 and 2, each of the electrically-conductive traces 34 may extend from the distal end portion 14 of the shaft 12 (*e.g.,* from a point axially-coincident with a respective one of the electrodes 18, 20) to the proximal end portion 16 of the shaft 12, in an embodiment. Each trace 34 may extend over substantially the entire length of the shaft 12, in an embodiment. For example, each trace 34 may extend over 90% or more of the length of the catheter shaft 12. In an embodiment, one or more of the traces 34 may include one or more interruptions and/or discontinuities. For example but without limitation, a distal portion of a trace 34 may extend from the distal end portion 14 of the shaft 12, be electrically coupled with a distal end of a flex circuit, such as a flex circuit as illustrated and described in U.S. patent application publication no. 2012/0172842, and a proximal portion of the trace 34 may be electrically coupled with a proximal end of the flex circuit and may continue extending proximally to the proximal end portion 16 of the shaft 12.

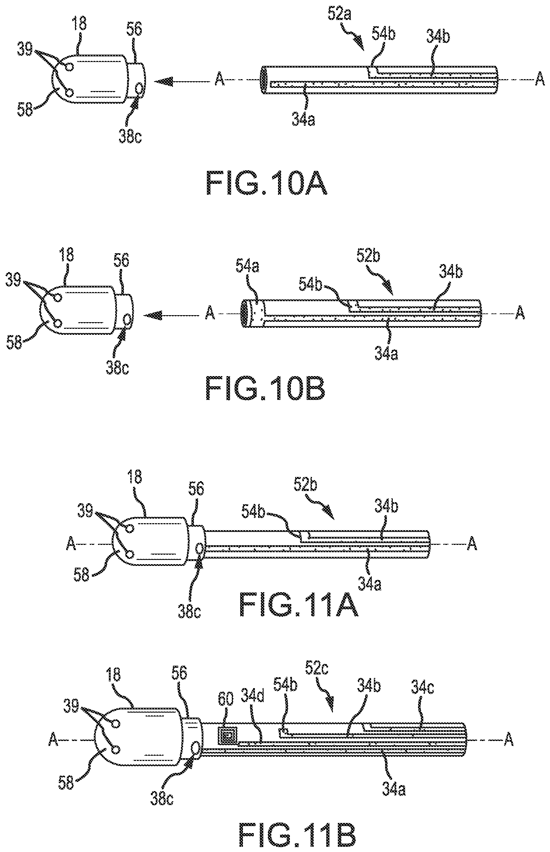

Figures 3A-14 illustrate several stages of buildup in a method of manufacturing and assembling an embodiment of the catheter shaft 12 illustrated in Figures 1 and 2. More particularly, Figures 3A-9 illustrate an exemplary method of depositing one or more electrically-conductive traces 34 on an inner tube 32 of a catheter shaft 12, and Figures 10A-14 illustrate further steps in an exemplary method of manufacturing and assembling a catheter shaft 12 that may include the inner tube 32 with electrically-conductive traces 34. It should be understood that the steps and methods shown and described herein are exemplary in nature only. Steps may be added, altered, and/or omitted without departing from the scope of the instant disclosure.

Referring to Figures 3A-9, a method of depositing one or more electrically-conductive traces 34 on an inner tube 32 may begin with providing an inner tube 32. Figure 3A is an isometric view of an intermediate portion 40 of the inner tube 32, and Figure 3B includes a side view of the intermediate portion 40 and an end view of the inner tube 32. The inner tube 32 may have an inner diameter and an outer diameter defining a wall 42, and may define an inner lumen 44.

In an embodiment, the inner tube 32 may have an inner diameter of about 0.032 inches, an outer diameter of about 0.0365 inches, a wall thickness of about 0.0045 inches, and a length of about 60 inches, 1 inch being 2,54 cm. The inner tube 32 may be an extruded polymer, in an embodiment. Alternatively, the inner tube may be formed from a flat substrate, which is rolled and bonded to form a tube. The rolling and bonding may happen before or after other process steps of the various methods illustrated and/or described herein.

The inner tube 32 may comprise, for example but without limitation, a polymer, such as polyimide. Additionally or alternatively, the inner tube 32 may be or may include polyethylene-naphthalate (PEN), such as a PEN film. For example, the inner tube 32 may be or may include a PEN film commercially available under the trade name Teonex®, such as Teonex® Q65FA or Teonex® Q83. Additionally or alternatively, the inner tube 32 may be or may include polyethylene terephthalate (PET), such as a PET film. For example, the inner tube 32 may be or may include a PET film commercially available under the trade name Melinex®, such as Melinex® ST506 or Melinex® ST504.

Additionally or alternatively, the inner tube 32 may comprise another material having material characteristics suitable for one or more of heightened processing temperatures temperatures (*e.g.,* capable of withstanding temperatures involved in melt processing further layers of a catheter shaft), for the materials deposition methods and steps described or referenced herein, and for a minimum thickness.

Additionally, the materials comprising the inner tube 32 may be appropriate for safely transmitting fluid (*i.e.,* in a biologically-compatible manner) through the lumen 44 of the inner tube 32. Thus, the lumen 44 may act as a fluid lumen in the finished device for, *e.g.,* the flow of irrigation fluid to the tip of the device.

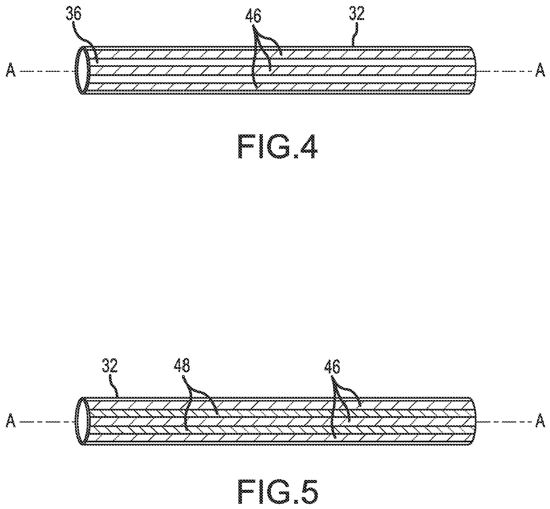

Referring to Figure 4, one or more masks 46 may be placed on the exterior surface 36 of the inner tube 32. The masks 46 may comprise materials and processes known in the art such as, for example, those offered commercially by Enthone, Inc. of West Haven, Connecticut.

The masks 46 may be placed on the exterior surface 36 of the inner tube 32 to define the line width (*i.e.,* width of a given trace 34), spacing between traces 34, and pattern requirements of a particular application. In an embodiment, the line width of an individual trace 34 may be between about 25 µm and about 100 µm. The masks 46 may be placed on every portion of the outer surface 36 of the inner tube 32 where an electrically-conductive trace 34 is not desired (*i.e.,* negative masking), in an embodiment. Alternatively, the masks 46 may be placed on the portions of the outer surface 36 of the inner tube 32 where an electrically-conductive trace 34 is desired (*i.e.,* positive masking), in an embodiment. The remainder of the description herein will be with respect to an embodiment employing negative masking, but it should be understood that this is for ease of description only, and is not limiting.

Referring to Figure 5, a seed layer 48 may then be deposited on the exposed (*i.e.,* non-masked) portions of the outer surface 36 of the inner tube 32. The seed layer 48 may comprise, for example and without limitation, copper or another suitable metal. The seed layer 48 may be deposited through, for example, chemical vapor deposition (CVD), physical vapor deposition (PVD), electrografting, and/or known "wet" methods of deposition. Electrografting may be performed, for example, according to a technique described in Frederic Raynal (2012), "Integration of Electrografted Layers for the Metallization of Deep Through Silicon Vias," Electroplating, Prof. Darwin Sebayang (Ed.), ISBN: 978-953-51-0471-1.

In addition to a seed layer 48, a tiecoat layer may be deposited. The tiecoat layer may be deposited in substantially the same manner as the seed layer 48, in an embodiment (*e.g.,* according to chemical vapor deposition (CVD), physical vapor deposition (PVD), etc.). The tiecoat layer may be deposited before the seed layer 48. The tiecoat material may comprise a chromium-based or nickel-based alloy, in an embodiment. The tiecoat layer may improve the adhesion of electrically-conductive materials to the inner tube 32.

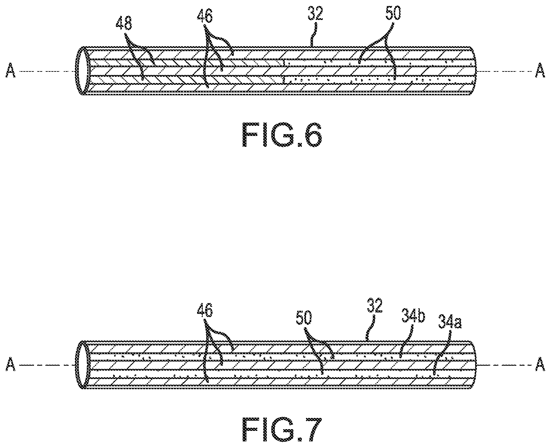

A conductive layer 50 may then be deposited on top of the seed layer 48, as shown in Figures 6 (illustrating partial conductive layer 50 deposition) and 7 (illustrating completed conductive layer 50 deposition). The conductive layer 50 may comprise a conductive metal such as, for example and without limitation, copper, nickel, and/or gold. The conductive layer may be deposited through electroplating, electroless deposition, CVD, and/or PVD, for example.

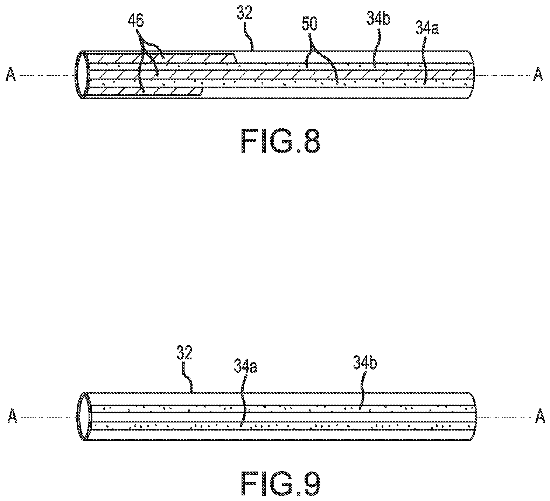

The mask layer 46 may be removed, as illustrated in Figures 8 (illustrating partial mask removal) and 9 (illustrating complete mask removal). The masks 46 may be removed using conventional mask removal materials (*e.g.,* solvents) and processes, which materials and processes may be selected according to the materials used for the masks 46.

In some embodiments, the mask layer 46 may be left intact on the inner tube 32. For example, the mask layer 46 may be left intact where positive masking is employed. The mask layer 46 may also be left intact (*i.e.,* in an embodiment employing negative masking) to serve as a dielectric between adjacent traces 34. In particular, the mask layer 46 may be left intact as a dielectric layer in embodiments in which the space between traces 34 in relatively small.

In an additional process step, a coating layer may be deposited over the traces 34, remaining masks 46, and/or exposed portions of the outer surface 36 of the inner tube 32. For example, a coating layer comprising polymer, such as, for example only, that sold under the trade name PARYLENE HT, available from Specialty Coating Systems, Inc. of Indianapolis, Indiana may be deposited. The coating layer may be deposited through CVD and/or PVD, in an embodiment. The coating layer may be provided, for example, as a dielectric and/or to prevent physical damage to the traces 34 during further manufacturing and assembly steps of a catheter shaft 12 including the inner tube 32, as well as during use of the finished catheter shaft 12.

After the mask layer 46 is removed (or with the mask layer 46 still intact), the result may be an inner tube 32 on which one or more electrically-conductive traces 34 are disposed. The steps illustrated in Figures 3A-9 may be used to deposit electrically conductive traces 34 in a desired pattern. Deposited traces 34 may include, for example and without limitation, longitudinal straight lines, circumferential contact pads, serpentine patterns, spiral patterns, etc. Such patterns may be deposited to provide an electrical infrastructure through which one or more sensors (such as the electrodes 18, 20, see Figures 1 and 2) may be electrically connected to external systems (*e.g.,* through an electromechanical connector in the handle disposed at the proximal end of the shaft).

Deposited traces 34 may also be used to form sensors themselves. For example, deposited traces 34 may be used to form sensors such as, for example only, GPS antennas, coils for use in electromagnetic positioning systems, etc.

As an alternative to the process steps illustrated in Figures 4-9, electrically-conductive traces 34 may be deposited on the inner tube 32 by printing. Printed ink traces 34 may comprise copper or silver ink, in an embodiment. Traces 34 may be printed with equipment and processes known in the art such as, for example, equipment available from Optomec, Inc. of Albuquerque, New Mexico.

In an alternative process, electrically-conductive traces 34 may be printed on a non-cylindrical inner structure, such as a flat substrate, substantially according to the steps illustrated and described herein. The non-cylindrical inner structure may then, in an embodiment, be formed into a tube or other shape with adhesive and/or other fastening means. If formed into a tube or tube-like structure, such inner layer may thereafter serve as an inner tube 32 for later manufacturing steps, assembly steps, and uses described herein.

In an exemplary embodiment in which the inner tube 32 is formed from polyimide, the polyimide may be pretreated in a plasma pretreatment process before deposition of electrically-conductive and other materials. The plasma pretreatment may improve adhesion of the deposited materials relative to a non-pretreated polyimide.

Figures 10A-14 illustrate further steps in a method of manufacturing and assembling a catheter shaft 12 that may include the inner tube 32 with electrically-conductive traces 34. Various embodiments of a distal end portion 52a, 52b, 52c of various embodiments 32a, 32b, 32c of the inner tube 32 are shown in Figures 10A-12.

As shown in Figures 10A and 10B, a tip electrode 18 may be provided. As shown in Figure 10A, the tip electrode 18a may be configured to be electrically coupled with the distal tip of a longitudinal trace 34a, in an embodiment. Additionally or alternatively, as shown in Figure 10B, the inner tube distal end portion 52b may include, and the tip electrode 18b may be configured to be electrically coupled with, a circumferential bonding pad 54a. The circumferential bonding pad 54a may be deposited according to the steps and methods described above in conjunction with Figures 3A-9. The circumferential bonding pad 54a may include a circumferential dimension that is larger than the longitudinal dimension of the bonding pad 54a. In an embodiment, the bonding pad 54a may extend around the entire circumference of the inner tube distal end portion 52b. Alternatively, a bonding pad 54 may extend around less than the entire circumference of the inner tube distal end portion 52a, 52b, 52c. For example, as shown in both Figures 10A and 10B, a bonding pad 54b may be provided that does not extend around the entire circumference of the inner tube distal end portion 52a, 52b. It should be noted that the bonding pads 54a, 54b, and similar structures may be described herein collectively as the bonding pads 54, or individually as a bonding pad 54.

The tip electrode 18a, 18b may include a neck portion 56 and a body portion 58, in an embodiment. The neck portion 56 may include a bore 38c, in an embodiment. The bore 38c may be formed by laser drilling, mechanical drilling, and/or another bore-formation technique. The bore 38c may extend into the body portion 58 of the tip electrode 18, substantially orthogonal to the longitudinal axis of the final catheter shaft. In other embodiments, the neck portion 56 of the tip electrode 18 may lack a bore 38c.

The inner tube distal end portion 52a, 52b, 52c may be inserted into a cavity in the proximal end of the tip electrode 18, in an embodiment, as shown in Figures 11A and 11B. The cavity may extend through the neck portion 56 and at least partially into the body portion 58, in an embodiment. The inner tube distal end portion 52a, 52b, 52c may be inserted such that a portion of an electrically-conductive trace 34 is circumferentially coincident with the bore 38c in the neck portion 56.

The inner tube 32 may be rigidly coupled (*e.g.,* bonded) with the tip electrode 18 using an adhesive, in an embodiment. For example, the inner tube 32 may be rigidly coupled with the tip electrode 18 with an electrically-insulative adhesive. Alternatively, the inner tube 32 may be rigidly coupled with the tip electrode 18 with an electrically-conductive adhesive. In an embodiment, such adhesive may provide an electrical coupling between one or more traces 34 on the inner tube 32 and the tip electrode 18.

Following coupling of the tip electrode 18 with the inner tube 32, the bore 38c in the neck portion 56 of the tip electrode 18 may be cleaned out (*e.g.,* to remove electrically non-conductive adhesive and/or other debris). In an embodiment, the bore 38c may be cleaned out through laser drilling. In particular, the bore 38c may be cleaned out if an electrically-insulative adhesive is used to couple the tip electrode 18 with the inner tube 32.

The bore in the neck portion of the tip electrode 18, if provided, may be filled with an element that electrically couples the electrically-conductive trace 34a with the tip electrode 18. For example, in an embodiment, the bore 38c may be filled with an electrically-conductive adhesive.

As shown in Figure 11B, and as noted above, in an embodiment, electrically-conductive traces 34 may be deposited on the inner tube 32 so as to form a sensor. For example only, electrically-conductive traces may be deposited in a spiral formation to form an antenna 60 that may be used, for example only, to receive GPS signals.

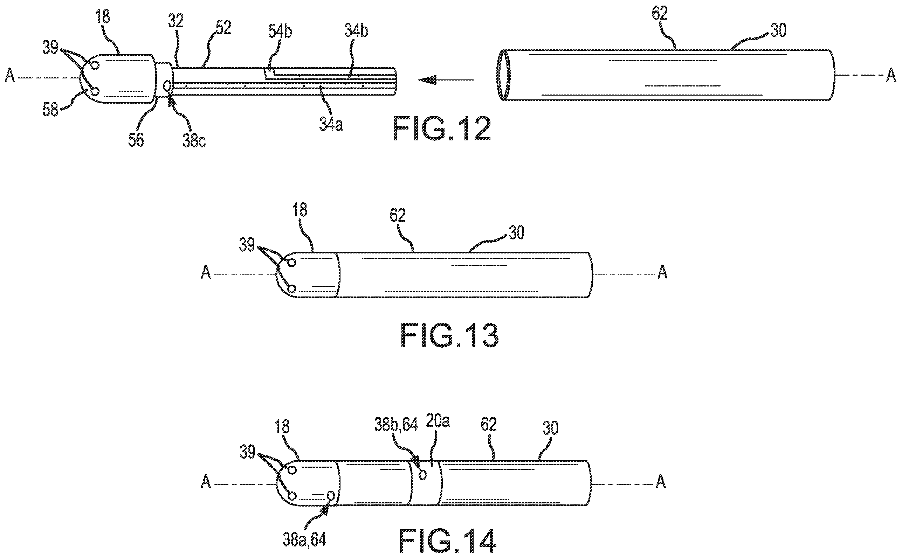

Referring to Figure 12, an outer tube 30 may be provided (Figures 12-14 show a distal end portion 62 of the outer tube 30). The outer tube 30 may comprise a polymer such as, for example and without limitation, polyether block amide (PEBA). The outer tube 30 may be pre-formed (*e.g.,* before being placed over the inner tube 32) to a desired shape and dimensions (*e.g.,* desired inner diameter, outer diameter, and length). The outer tube 30 may be pre-formed by extrusion or melt processing on a separate mandrel, for example. Alternatively, the outer tube 30 may be melt-processed on the inner tube 32 (and/or on another layer of the catheter shaft 12) to obtain desired dimensions. The outer tube 30 may have an outer diameter that is substantially the same as the outer diameter of the tip electrode 18, in an embodiment.

Referring to Figure 13, the outer tube 30 may be placed over the inner tube 32 and the neck portion 56 of the tip electrode 18. The outer tube 30 may be rigidly coupled (*e.g.,* bonded) to the tip electrode 18, in an embodiment. For example, the inner surface of the outer tube 30 may be rigidly coupled to the outer surface of the neck portion 56 of the tip electrode 18 with electrically-insulative adhesive or electrically-conductive adhesive.

Referring to Figure 14, one or more ring electrodes 20 may be placed over the outer tube 30 (one such ring electrode 20a is shown in Figure 14). Each ring electrode 20 may be placed to be axially-coincident with a portion of a respective electrically-conductive trace 34 on the inner tube 32. For example, each ring electrode 20 may be placed such that a portion of each ring electrode 20 is axially coincident with a bonding pad 54. Each ring electrode 20 may be rigidly coupled with the outer tube 30 such as, for example only, with adhesive.

One or more bores 38 may be made in each electrode 18, 20, in an embodiment. Each bore 38 may be made by, for example only, laser drilling and/or mechanical drilling. Each bore 38 may be substantially orthogonal to the longitudinal axis A of the shaft, and may extend from an outer surface of the electrode 18, 20, through the electrode 18, 20, and, for the ring electrodes 20, through any portion of the outer tube 30 that is radially inward of the electrode 20. Each bore 38 may thus provide a hole from the outer surface of the electrode 18, 20 to a portion of an electrically-conductive trace 34. A bore 38 may be circular, in an embodiment, or may have some other shape, in another embodiment.

Through a respective bore 38, an element may be provided to electrically couple each electrode 18, 20 with a respective electrically-conductive trace 34. In an embodiment, for example, each bore 38 may be filled with electrically-conductive adhesive 64.

In an embodiment, one or more of the bores 38 may be formed in the electrodes 18, 20 and/or the outer tube 30 before assembly of the inner tube 32, tip electrode 18, outer tube 30, and ring electrodes 20. Accordingly, in an embodiment, a part of the assembly process may involve placing the inner tube 32, tip electrode 18, outer tube 30, and/or ring electrodes 20 to line up bores 38 and traces 34 with each other.

The catheter 10 may operate with a variety of catheter systems such as visualization systems, mapping systems, and navigation support and positioning systems (*i.e.,* for determining a position and orientation (P&O) of a flexible elongate member or other medical device). For example, the catheter 10 may find use with a visualization, mapping, and navigation system. Such a "navigation system" may comprise an electric field-based system, such as, for example, an EnSite™ Velocity™ cardiac electro-anatomic mapping system running a version of EnSite™ NavX™ navigation and visualization technology software commercially available from St. Jude Medical, Inc., of St. Paul, Minnesota and as also seen generally by reference to U.S. Patent Nos. 7,263,397 and 7,885,707. In other exemplary embodiments, the navigation system may comprise systems other than electric field-based systems. For example, the navigation system 70 may comprise a magnetic field-based system such as the Carto™ system commercially available from Biosense Webster, and as generally shown with reference to one or more of U.S. Patent Nos. 6,498,944; 6,788,967; and 6,690,963. In another exemplary embodiment, the navigation system may comprise a magnetic field-based system based on the MediGuide™ technology available from St. Jude Medical, Inc., and as generally shown with reference to one or more of U.S. Patent Nos. 6,233,476; 7,197,354; and 7,386,339. In yet another embodiment, the navigation system may comprise a combination electric field-based and magnetic field-based system, such as, for example and without limitation, the system described in pending U.S. Patent Application No. 13/231,284, or the Carto™ 3 system commercially available from Biosense Webster, and as generally shown with reference to U.S. Patent No. 7,536,218. In yet still other exemplary embodiments, the navigation system may comprise or be used in conjunction with other commonly available systems, such as, for example and without limitation, fluoroscopic, computed tomography (CT), and magnetic resonance imaging (MRI)-based systems.

*Elongate Medical Device Incorporating Coil Sensor and Flexible Substrate.* Referring again to Figure 1, in a second aspect of the present disclosure, an alternate embodiment of the catheter 10 may include a sensor disposed inside of a flexible substrate. Such a sensor will be described in this second aspect of the disclosure as an electromagnetic coil sensor, but this is for convenience only. A variety of sensors may find use in the arrangement of this second aspect of the disclosure, in embodiment. Furthermore, in an embodiment in which the catheter 10 includes an electromagnetic coil sensor as set forth in this second aspect of the disclosure, such a coil sensor may be provided in addition to or instead of one or more of the electrodes illustrated in Figure 1.

Referring to Figure 15, the shaft of the catheter (designated shaft 12', where prime notation indicates an alternate embodiment having similar characteristics) may include an elongate, tubular member 70 and a tip 72. Member 70 is flexible or deformable and configured for movement within the body of the patient. Member 70 also defines one or more lumens configured to house conductors 74, 76 and steering wires and to allow fluids (*e.g.,* irrigation fluid) to pass. Member 70 may include a tubular, polymeric inner liner 78, a braided wire layer 80 for torque transfer, and an outer polymeric jacket 82. Liner 78 may be made from a polymeric material such as polyfluoroethylene (PTFE) including PTFE sold under the registered trademark "TEFLON" by E.I. DuPont de Nemours & Co. Corp, polyether block amides, nylon or thermoplastic elastomers such as the elastomer sold under the registered trademark "PEBAX" by Arkema, Inc. Braided wire layer 80 may be configured to provide appropriate levels of pushability, torqueability, flexibility, and kink resistance to shaft 12'. Braided wire layer 80 may be formed from stainless steel wire, and may be flat wire (wire having a cross-section that, when taken along the wire's longitudinal axis and measured along two orthogonal axes, is substantially rectangular) arranged in various braid patterns including one-over-one (involving at least two wires) or two-over-two (involving at least four wires) crossover patterns. The wire may be coated with a layer of an insulating material. The wire braid may be directly wound about liner 78 or placed on a core that is slid over liner 78. Jacket 82 may be made from a polymeric material such as polyfluoroethylene (PTFE) including PTFE sold under the registered trademark "TEFLON" by E.I. DuPont de Nemours & Co. Corp, polyether block amides, nylon or thermoplastic elastomers such as the elastomer sold under the registered trademark "PEBAX" by Arkema, Inc. and may be extruded over braided wire layer 80. Additional details regarding several exemplary catheter constructions may be found in commonly assigned U.S. Patent No. 7,914,515. Distal tip 72 may be received at a distal end 84 of member 70. Tip 72 may be made from a material or materials that are relatively rigid and may comprise, or may be configured to support, an electrode (*e.g.,* such as one of the electrodes 20 illustrated in Figure 1).

With continued reference to Figure 15, an electronic subassembly 86 may be provided to perform any of a variety of functions performed by electronic components in a given medical device. In accordance with one embodiment of the present teachings, electronic subassembly 86 may be provided for use in determining the position of the catheter 10, and particularly distal end 14' or distal tip 72 of catheter 10. The electronic subassembly 86 may alternatively be used to perform various functions associated with the catheter 10 including, for example, controlled delivery of ablation current to a tip electrode, electrogram sensing, temperature sensing and signal processing or conditioning. It should further be understood that the functionality of the subassembly 86 will depend on the nature of the medical device and that subassembly 86 may therefore perform different functions where the device is not an ablation catheter. Although one electronic subassembly 86 is shown in the illustrated embodiment, it should be understood that multiple electronic subassemblies 86 may be used to perform the same, related, or different functions within the catheter 10. In the illustrated embodiment, subassembly 86 is illustrated as being disposed in the distal portion 14' of the shaft 12'. Alternatively, electronic subassembly 86 may be disposed in any portion of the shaft 12' (including in an orifice in distal tip 72 or other structure forming a lumen within shaft 12' proximal to tip 72). Subassembly 86 may include a flexible substrate 88, one or more electronic devices 90 mounted on the substrate 88 and one or more conductors 92, 94 extending from substrate 88.

Flexible substrate 88 may provide structural support and may serve as a mechanical and electrical connector for electronic device 90 and conductors 92, 94. Substrate 88 may be made from any insulative and relatively flexible material, such as polyimides or polyethylene terephthalate (PET). Referring to Figure 15, in one state, substrate 88 may be generally rectangular in shape and have a length 96 and a width 98. Substrate 88 may define opposite sides 100, 102 with side 100 comprising an interior side and side 102 comprising an exterior side upon final assembly as described below. Substrate 88 may further define edges extending between sides 100, 102, including edges 104, 106 disposed on opposite sides of substrate 88. Edges 104, 106 may be perpendicular to sides 100, 102, or may extend at a non-perpendicular angle between sides 100, 102. Substrate 88 may define one or more conductive areas 108, 110 (*i.e.,* contact pads) on interior side 100 and one or more conductive areas 112, 114 on exterior side 102. Substrate 88 may further define conductive paths 116, 118 between conductive areas 108, 112 and 110, 114, respectively. In the illustrated embodiment, conductive areas 108, 110, 112, 114 are disposed at a proximal end 120 of substrate 88. It should be understood, however, that one or more of conductive areas 108, 110, 112, 114 could be located at a distal end 122 of substrate 88 or at a location between ends 120, 122. Further, conductive areas 108, 110, 112, 114 may be disposed on any surface of substrate 88 including any edge extending between sides 100, 102. Also in the illustrated embodiment, conductive areas 108, 112, and 110, 114 are aligned such that conductive paths 116, 118, extend the shortest possible distance between areas 108, 112, and 110, 114, respectively. It should be understood, however, that areas 108, 110, 112, 114 could be located at various locations on substrate 88 with paths 116, 118 taking a straight or circuitous route through substrate 88 to electrically couple areas 108, 112, and 110, 114, respectively. Conductive areas 108, 110, 112, 114 may be made from conductive materials such as copper and may have a surface finish such as electroless nickel/gold, silver, etc.

One of ordinary skill in the art will understand that conductive paths 116, 118 can be formed by (for example and without limitation) constructing through vias and/or blind vias through the flexible substrate 88. In one embodiment, conductive paths 100, 102 are formed by creating through vias between conductive areas 108, 112, and 110, 114 and filling the through vias with a conductive material, such as copper. Thus, conductive paths 116, 118 can be formed during the formation/manufacture of the flexible substrate 88 itself (*e.g.,* during plating). In another embodiment, conductive paths 116, 118 are formed by laser-drilling through the flexible substrate 88 to conductive areas 108, 110, 112, 114 and, thereafter, filling the drilled holes with conductive paste. The lead wires from electronic device 90 and/or the conductors 92, 94 may be electrically connected to conductive paths 116, 118 and conductive areas 108, 110, 112, 114 during the curing of the conductive paste. In some embodiments, some or all of conductive areas 108, 110, 112, 114 can be made from conductive paste.

Device 90 may be a position sensor provided for use in determining the position of the distal portion of catheter 10 within a coordinate system and within a patient's body, in an embodiment. As discussed hereinabove, however, the function of device 90 may vary depending on the nature of the medical device in which subassembly 86 is used. Further, although the illustrated embodiment shows a single device 90 on substrate 88, it should be understood that multiple devices 90 may be mounted to substrate 88. Device 90 may comprise a sensor such as an electromagnetic field detector, which may include a coil assembly 124 that includes a coil 126. When the distal tip 72 of catheter 10 is moved within a magnetic field, the current induced in coil 126 will vary and the coil 126 will generate a signal indicative of the position of distal tip 72 of catheter 10. Although a single coil 126 is shown in the illustrated embodiment, multiple coils may be mounted on substrate 88 or on different substrates as part of different subassemblies to provide a determination of the position of tip 72 of catheter 10 in three-dimensional space. The coil 126 may extend along the entire length 96 or width 98 of substrate 88 or only a portion of the length 96 or width 98. In the illustrated embodiment, the coil 126 is disposed in the center of substrate 88, but it should be understood that the location of the coil 126 on substrate 88 may vary. The coil 126 may comprise a continuous wire wound in a helix, with a plurality of turns of wire, each wrapped about an axis that may be parallel or coincident with the axis of the completed catheter 10. Coil assembly 124 may further include a magnetic core 128 about which the coil 126 may be wrapped. The coil assembly 124 may include leads 130, 132 at either or both ends of coil 126. Leads 130, 132 may be coupled to conductive areas 108, 110, respectively, by soldering and may each have a surface area. Coil assembly 124 may further be mounted to substrate 88 by using an adhesive such as a nonconductive epoxy or polyurethane on one or both of substrate 88 and coil assembly 124 and curing the adhesive in an oven.

Conductors 92, 94 are provided to transfer electrical signals among components within catheter 10 and, in particular, between device 90 and an external device or system (*e.g.,* a position and navigation system) or other signal processing and conditioning circuits. Conductors 92, 94 may comprise wires or cables connected to and extending from substrate 88 to the proximal end 16 (see Figure 1) of shaft 12'. Alternatively, conductors 92, 94 may comprise printed traces formed on the surface of a lumen extending through shaft 12' as discussed in greater detail above.

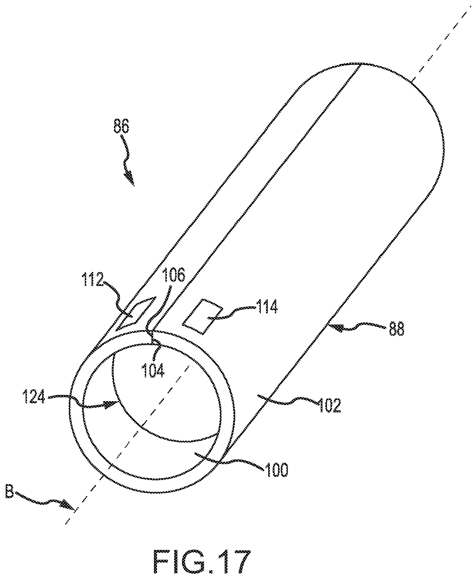

Referring now to Figure 17, electronic assembly 86 is shown in a deformed state. Due to its flexibility, flexible substrate 88 can be deformed, as illustrated in Figure 17. In an embodiment, substrate 88 may be deformed about an axis, such as longitudinal axis B, and shaped into a cylinder (*i.e.,* tube) that at least partially encloses and, in the illustrated embodiment, circumferentially surrounds, electronic device 90. In the illustrated embodiment, edges 104, 106 are brought together and bonded with an adhesive such as an ultraviolet cure adhesive. It should be understood that flexible substrate 88 could alternatively be deformed by bringing the edges extending between edges 104, 106 together such that substrate 88 is deformed about an axis perpendicular to axis B and would surround the axial ends of coil assembly 124. Further, although the illustrated embodiment shows edges 104, 106, aligned along their lengths such that the end face of substrate 88 is annular in shape, it should be understood that edges 104, 106 may only partially overlap such that the end face of substrate 88 takes on a more helical shape. In an alternative embodiment, substrate 88 may be deformed such that edges 104, 106 extend past one another and a portion of sides 100, 102 overlap. In such an embodiment, the bonding would occur in the overlapped area between sides 100, 102, and conductive paths 116, 118 may extend into and/or through the overlapped area. Substrate 88 can also be deformed into various shapes and formations, such as a cone. Because substrate 88 at least partially encloses device 90, substrate 88 protects device 90 from external forces and provides a more robust subassembly 86. Further, conductive areas 108, 110, 112, 114 remain easily accessible for connection thereto by device 90 and conductors 92, 94. Conductors 92, 94 may each have a distal end (for connection to subassembly 86), each end with its own surface area. In an embodiment, conductive areas 108, 110, 112, 114 each have a surface area that is greater than the surface areas of the distal ends of conductors 92, 94 and the surface areas of leads 130, 132 of coil assembly 124 (*e.g.,* to strengthen the respective bonds). Although the flexible substrate 88 is illustrated as being deformed to at least partially enclose electronic subassembly 86 (by coupling edges of the substrate 88), one of ordinary skill in the art would understand that the substrate 88 may be pre-formed (*e.g.,* by extrusion) to assure a shape that will at least partially enclose subassembly 86 upon assembly. In such an embodiment, substrate 88 may be a unitary structure without joints between portions of substrate 88. Furthermore, one of ordinary skill in the art would understand that the flexible substrate 88 may not be deformed and/or that the electronic subassembly 86 may not be enclosed. For example and without limitation, the flexible substrate 88 may remain undeformed as illustrated in the embodiment of Figure 15.

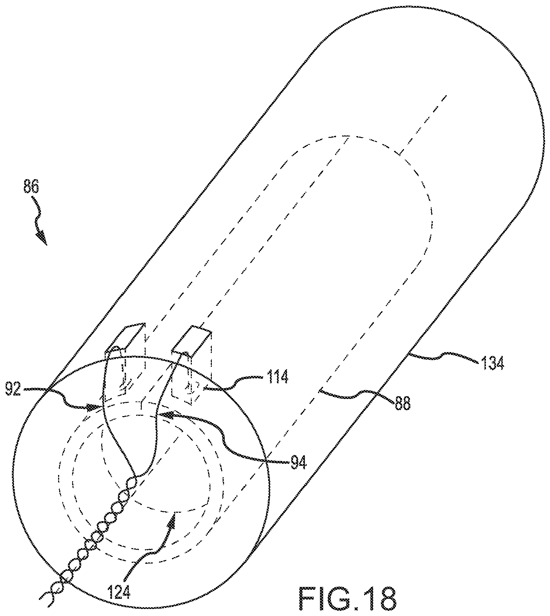

Referring now to Figure 18, subassembly 86 is shown in an encapsulated state. One of ordinary skill in the art would understand that encapsulation is optional and that electronic subassembly 86 may remain unencapsulated as illustrated in Figure 15. Subassembly 86 may be encapsulated with an electronics molding compound 134. Because electronics molding compounds 134 are silica-filled thermoset resin systems, they provide high material strength and durability, thermal conductivity, and resistance to dielectric breakdown. Therefore, compound 134 provides more protection and robustness for post-encapsulation processing and assembly. It should be understood that other alternative molding materials and methods may be used, such as silicone liquid injection molding (LIM) and insert injection molding using thermoplastic resins (*e.g.,* polycarbonate). The desired thickness of compound 134 may also vary depending on the catheter size and desired protection of electronic subassembly 86. Additionally, encapsulated electronic subassembly 86 may be generally cylindrical in shape. However, encapsulated electronic assembly 86 may be shaped in a variety of forms. Following encapsulation, conductive areas 112, 114 may be exposed through compound 134 so that conductors 92, 94 may be connected thereto. Conductors 92, 94 may be soldered to conductive areas 112, 114 using reflowed solder paste.

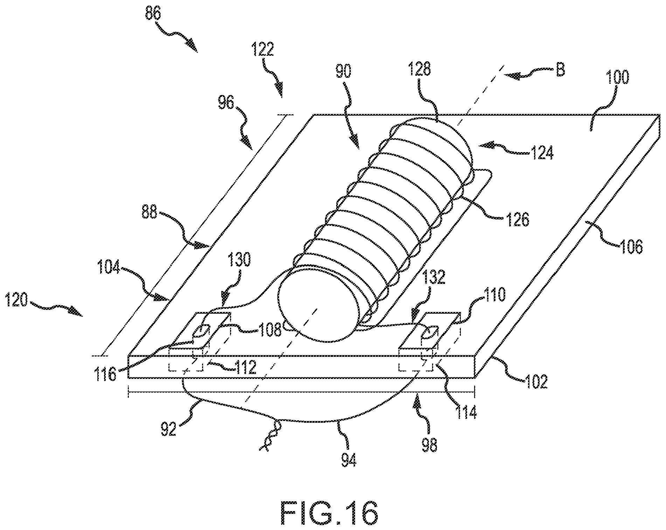

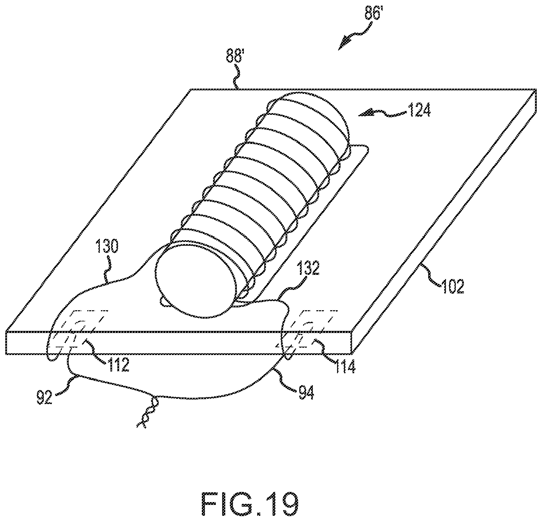

Referring now to Figures 19-21, an alternative embodiment of an electronic subassembly 54' is shown prior to deformation, in a deformed state and in an encapsulated state. As with the embodiments of Figures 16-18, deformation of flexible substrate 88 and encapsulation of subassembly 54' are optional. Subassembly 54' is similar to subassembly 54, but the leads 130, 132 of coil assembly 124 are connected to conductive areas 112, 114 on exterior side 102 of substrate 88'. Although leads 130, 132 are illustrated as extending around an edge of flexible substrate 88', it should be understood that leads 110, 112 could alternatively extend through substrate 88' directly to conductive areas 112, 114 via bores in substrate 88'. Conductors 92, 94 are likewise connected to conductive areas 112, 114. As a result, subassembly 86' eliminates several conductive areas and conductive paths between areas found in substrate 88 of subassembly 86.

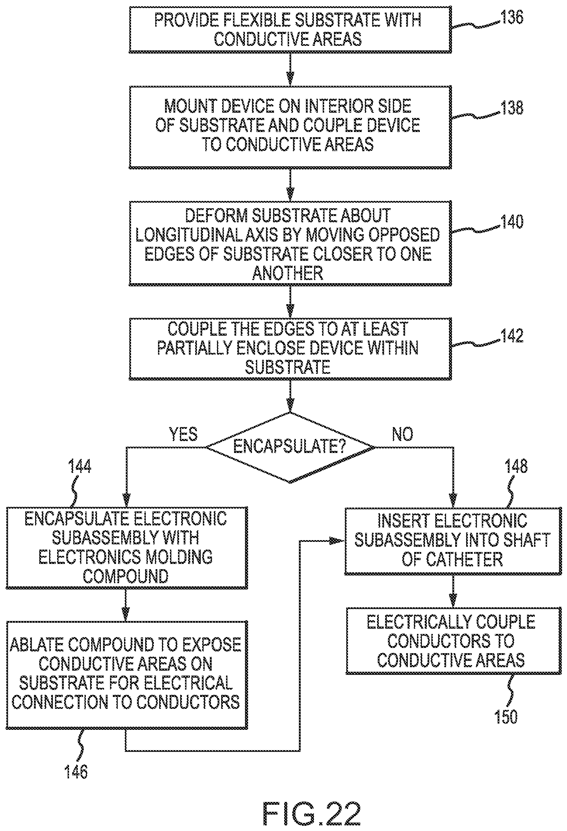

Referring now to Figure 22, one or more exemplary methods for fabricating a medical device 10 are illustrated. The method may begin with the process 136 of providing a flexible substrate 88 or 88'. As set forth hereinabove, substrate 88 may include conductive areas 108, 110 on the interior side 100 of substrate 88, conductive areas 112, 114 on the exterior side 102 of substrate 88 and conductive paths 116, 118 extending between the conductive areas 108, 112 and 110, 114, respectively. Substrate 88' may include conductive areas 112, 114 on the exterior side 102 of substrate 88'.

The method may continue with the process 138 of mounting device 90 on the interior side 100 of substrate 88 or 88' and coupling device 90 to one or more conductive areas such as conductive areas 108, 110 on substrate 88 or conductive areas 112, 114 on substrate 88'. Process 138 may include several subprocesses. In certain embodiments, an adhesive may be applied to one or both of substrate 88, 88' and device 90 to form a bonding area. The adhesive may comprise an ultraviolet (UV) adhesive. It should be understood, however, that other adhesives or epoxies may be used, such as nonconductive epoxies or polyurethane. After the adhesive is applied, the flexible substrate 88, 88' and device 90 may be bonded at the bonding area. Thereafter, the adhesive may be cured. Process 138 may further include electrically connecting device 90 to conductive areas 108, 110 of substrate 88 or 112, 114 of substrate 88'. Device 90 may be coupled to conductive areas 108, 110, 112, 114 by soldering leads 130, 132 to conductive areas 108, 110 on substrate 88 or 112, 114 on substrate 88'. In such an embodiment, reflowed solder paste may be applied to conductive areas 108, 110, 112, 114. Types of reflowed solder paste may include type 3 to 6 no-Pb solder pastes, such as Kester 520A SAC305. In accordance with another embodiment, conductive epoxy adhesives, such as Ablebond 2000, may be used to couple leads 130, 132 to conductive areas 108, 110 of substrate 88 or conductive areas 112, 114 of substrate 88'. Solder paste or conductive adhesive may be manually dispensed using a syringe, or it can be dispensed from automated equipment. After the paste or adhesive is applied, device 90 may be positioned such that device 90 contacts the solder paste. Therefore, substrate 88, 88' and device 90 may then be placed in a curing oven or reflow oven to cure the adhesive and/or reflowed solder paste.

The method may continue with the process 140 of deforming flexible substrate 88, 88' so as to move opposed edges, such as edges 104, 106, closer to one another. In accordance with one embodiment, process 140 may be performed using automated or semi-automated equipment. Substrate 88, 88' is deformed in such a way that it will at least partially enclose device 90. In the illustrated embodiment, for example, substrates 88, 88' may be deformed about axis B by bringing edges 104, 106 closer together to deform substrate 88, 88' into a cylindrical shape and circumferentially surround device 90. Although the illustrated embodiment, shows the process 140 of deforming substrate 88, 88' occurring after the process 138 of mounting device 90 to substrate 88, 88', it should be understood that processes 138, 140 could take place in the opposite order in whole or in part (*e.g.,* device 90 may be mounted to substrate 88, 88' prior to deformation, but leads 130, 132 may be coupled to conductive areas 108, 110, 112, 114 after deformation).

The method may continue with the process 142 of coupling edges 104, 106 to at least partially enclose device 90 within flexible substrate 88, 88'. Process 142 may include several subprocesses. An adhesive may be applied to one or both of edges 104, 106. The adhesive may comprise a UV cure adhesive so as to provide sufficient adhesive strength in a minimal amount of time. For example, suitable adhesives are Henkel Loctite 3913, 3971, and 3972 cured at 100mW/cm² flux. Thereafter, the edges 104, 106 may be overlapped such that the opposed edges 104, 106 are in contact with one another and the adhesive. Finally, the adhesive may be cured. It should be understood that edges 104, 106 may be coupled using other bonding mechanisms, such as mechanical fasteners. Further, edges 104, 106 may be coupled directly to one another or indirectly by coupling overlapping portions of sides 100, 102.

In certain embodiments, the method may optionally continue with the process 144 of encapsulating electronic subassembly 86, 86' with electronics molding compound 134. Process 144 may include several subprocesses Electronic subassembly 86, 86' may be placed in a mold cavity. Thereafter, compound 134 is dispensed into the mold cavity. Compound 134 can be made of Hitachi CEL 9200 (or 9700 series) mold compounds. It should be understood that other alternative molding materials and methods may be used, such as silicone liquid injection molding (LIM) and insert injection molding using thermoplastic resins (*e.g.,* polycarbonate). Suitable silicone LIM materials include Dow-Corning LSR & FLSR series materials. Once compound 134 has cured, electronic subassembly 86, 86' may be removed from the mold cavity.

If electronic subassembly 86, 86' is encapsulated, the method may continue with the process 146 of ablating compound 134 to expose conductive areas 112, 114 for electrical connection to conductors 92, 94. In accordance with one embodiment, such ablation may be laser ablation with laser energy tuned to ablate organic molding material disposed over conductive areas 112, 114.

Following process 146, or process 142 if no encapsulation is performed, the method may continue with the process 148 of inserting electronic subassembly 86, 86' into shaft 12' of catheter 10. In one embodiment, tip 72 of shaft 12' may include an orifice into which electronic subassembly 86, 86' may be inserted. Once inserted, the orifice may be filled with an epoxy such as polyurethane to secure electronic subassembly 86, 86'. Alternatively, adhesives may be used. In another embodiment, shaft 12' may include a lumen proximal to tip 72 through which conductors 92, 94 extend and/or irrigation fluid travels. In such an embodiment, electronic subassembly 86, 86' may be attached to structure forming the lumen.

The method may continue with the process 150 of electrically coupling conductors 92, 94 to conductive areas 112, 114. In accordance with one embodiment, conductors 92, 94 may be soldered to conductive areas 112, 114 In such an embodiment, reflowed solder paste may be applied to conductive areas 112, 114. Types of reflowed solder paste may include type 3 to 6 no-Pb solder pastes, such as Kester 520A SAC305. In accordance with another embodiment, conductive epoxy adhesives, such as Ablebond 2000, may be used to couple conductors 92, 94 with conductive areas 112, 114. Solder paste or conductive adhesive may be manually dispensed using a syringe, or it can be dispensed from automated equipment. Once conductors 92, 94 are coupled to conductive areas 112, 114, the solder paste (or conductive adhesive) may be cured in an oven.

A medical device and method for making the same in accordance with this second aspect of the disclosure may be advantageous relative to conventional devices and methods. For example, a more robust and compact sensor and a reliable connection between the sensor and proximally-extending conductors are provided, all while maintaining or improving the functionality of the device. In addition, the method for making the device is less complex and less expensive than conventional methods and results in smaller failure rates during post-fabrication testing.

*Elongate Medical Device Incorporating Coil Sensor and Full-Length Flexible Substrate.* As noted above in the second aspect of this disclosure, a flexible membrane may be wrapped around a sensor, such as a position sensor, such as an electromagnetic coil sensor, to provide structural support for the device and to protect the sensor. Further, as noted in both the first and second aspects of this disclosure above, a flexible tube (which may be formed with a wrapped flexible membrane, in an embodiment) may be used to provide electrical connectivity to the sensor, such as with electrical traces or vias on or through the tube. In an embodiment, as described below, the flexible substrate or tube may be lengthened to provide structural support and electrical connectivity over the entire length of the medical device.

Figures 23A-27 illustrate various steps in an exemplary method of assembling or manufacturing an elongate medical device. The medical device may be, in an embodiment, a guidewire. It should be understood, however, that one or more of the steps illustrated in an described with respect to Figures 23A-27 may be applied to assembly a medical device other than a guidewire. For example, one or more steps illustrated in or described with respect to Figures 23A-27 may be applied to assemble another elongate medical device, such as a catheter or introducer.

Figure 23A is a plan view of an initial stage of assembly of a guidewire assembly 160, and Figure 23B is an isometric view of a distal end portion 161 of the assembly 160. In reference to Figures 23A and 23B and subsequent figures, reference will be made to "the assembly 160." It should be understood that "the assembly 160" may be used to refer to the guidewire at various stages of its assembly, up to and including the completed guidewire, and thus may include few or many components, depending on the context in which it is used.

Referring to both Figure 23A and Figure 23B, the method of assembly may begin with providing a flat, flexible substrate 162 having a distal end portion 164 and a proximal end portion 166. Disposed on the substrate 162 may be one or more electrically-conductive traces 168 (two such traces 168a, 168b are illustrated, but any number of traces 168 may be provided). Each trace 168 may terminate at its distal end in a distal contact pad 170 (two such distal contact pads 170a, 170b are illustrated) and at its proximal end in a proximal contact pad 172 (two such proximal contact pads 172a, 172b are illustrated). Also disposed on the substrate may be an adhesive pad 174.

The substrate 162 may comprise, for example but without limitation, a polymer, such as polyimide. Additionally or alternatively, the substrate 162 may be or may include polyethylene-naphthalate (PEN), such as a PEN film. For example, the substrate 162 may be or may include a PEN film commercially available under the trade name Teonex®, such as Teonex® Q65FA or Teonex® Q83. Additionally or alternatively, the substrate 162 may be or may include polyethylene terephthalate (PET), such as a PET film. For example, the substrate 162 may be or may include a PET film commercially available under the trade name Melinex®, such as Melinex® ST506 or Melinex® ST504. The substrate 162 may comprise a single layer of material, in an embodiment. Alternatively, the substrate 162 may comprise a multi-layer construction, such as that set forth in the second aspect of this disclosure above (*i.e.,* the substrate 88).

The substrate 162 may have a longitudinal length, measured from the distal tip of its distal end portion 164 to the proximal top of its proximal end portion 166. The length of the substrate 162 may be substantially the same as (or, in an embodiment, slightly less than) the length of the finished elongate medical device. That is, the substrate 162 may extend from the distal end portion to the proximal end portion of the finished device. In an embodiment, the substrate 162 may have a length of one hundred (100) centimeters (cm) or more. Still further, in an embodiment, the substrate 162 may have a length of between 140 cm and 180 cm. Still further, in an embodiment, the substrate 162 may have a length of between 145 cm and 155 cm or 165 cm and 175 cm.

Each trace 168a, 168b may be a continuous trace of electrically-conductive material extending from a proximal pad 172a, 172b to a distal pad 170a, 170b. Each trace 168a, 168b may be applied to the substrate 162 according to one or more fabrication techniques generally used in semiconductor devices, in an embodiment. For example, the traces 168a, 168b may be deposited according to one or more techniques set forth above for the traces in the first aspect of this disclosure (*i.e.,* the traces 34a, 34b), in an embodiment.

In an embodiment, one or more of the traces 168a, 168b may include one or more interruptions and/or discontinuities. For example but without limitation, a distal portion of a trace 168a, 168b may extend from the distal end portion of the shaft, be electrically coupled with a distal end of a flex circuit, such as a flex circuit as illustrated and described in U.S. patent application publication no. 2012/0172842, and a proximal portion of the trace 168a, 168b may be electrically coupled with a proximal end of the flex circuit and may continue extending proximally.

The adhesive pad 174 may be provided to facilitate coupling a sensor with the substrate 162. The adhesive pad 174 may be, in an embodiment, a rectangular or other segment of material that is more adhesive than is the substrate 162. For example, the adhesive pad 174 may be an adhesive film. Additionally or alternatively, the adhesive pad 174 may be or may include a material that is more susceptible to bonding with a separate adhesive than is the rest of the substrate 162. For example, the adhesive pad 174 may be an area that has been plasma-treated or otherwise treated to improve wetting of the adhesive pad with a separate adhesive. In an embodiment, a separate adhesive may be applied between the adhesive pad 174 and a subsequently-placed sensor. For example, epoxy, polyurethane, acrylate, and/or methacrylate adhesive may be used. To prevent the flow of the bonding adhesive into unintended areas, the adhesive these materials can be applied and partially cured (*e.g.* b-staged), in an embodiment.

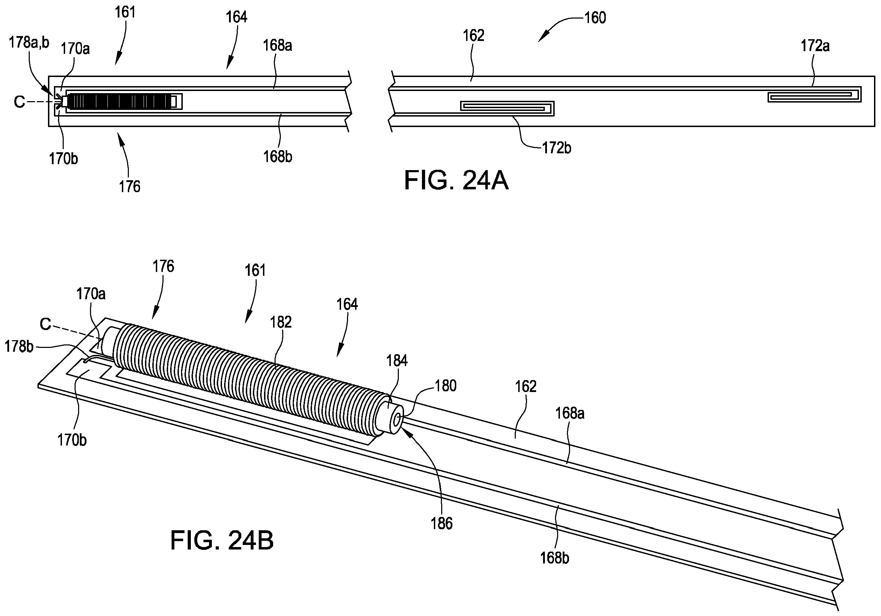

Figure 24A is a plan view of a further stage of build-up of the assembly 160, and Figure 24B is an isometric view of the distal end portion 161 of the assembly stage of Figure 24A. Referring to Figures 24A and 24B, a sensor 176 may be coupled with the distal end portion 164 of the substrate 162. The sensor 176 may be coupled, for example, with the adhesive pad 174. The sensor 176 may further be electrically coupled with one or more distal contact pads 170a, 170b, thereby electrically coupling the sensor 176 with one or more of the traces 168a, 168b. Such electrical coupling may be effected, for example, by soldering one or more wires 178 (two such wires 178a, 178b are illustrated) associated with the sensor 176 with the distal contact pads 170a, 170b.

In an embodiment, the sensor 176 may be a position sensor, such as a coil sensor, that may be used, for example, with an electromagnetic positioning system. In such an embodiment, the sensor 176 may comprise a core 180 and a coil 182 wrapped on the outer surface 184 of the core. The sensor 176 may be configured to return a signal that includes information that is indicative of a position and/or orientation of the sensor 176.

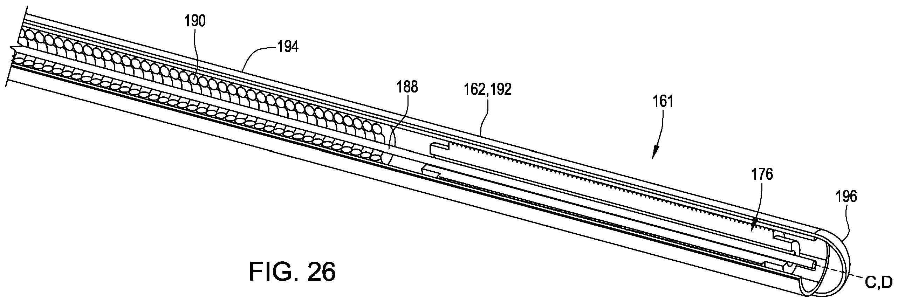

The core 180 may have a cylindrical shape having an outer surface 184. In the assembly, the sensor 176 may be arranged so that the longitudinal axis C of the core 180 is parallel with the longitudinal axis of the completed assembly 160 (longitudinal axis D, *see* Figure 26). Still further, in an embodiment, the longitudinal axis C of the core 180 may be substantially coincident with the longitudinal axis of the completed assembly. The core 180 may have a longitudinal lumen 186, in an embodiment. The lumen 186 may be parallel with one or both of the longitudinal axis C of the core 180 and the longitudinal axis D of the completed assembly 160, in an embodiment. Still further, in an embodiment, the lumen 186 may be disposed about one or both of the longitudinal axis C of the core 180 and the longitudinal axis D of the completed assembly 160. The core 180 may comprise a material having high magnetic permeability, such as iron or mu-metal, in an embodiment. Additionally or alternatively, the core 180 may comprise a polymer, such as polyimide.

The coil 182 may comprise a plurality of turns of electrically-conductive wire. Each turn of wire may be disposed about the longitudinal axis D of the completed assembly 160, in an embodiment. That coil may be configured to produce a signal induced by an external magnetic field (*e.g.,* such as from an electromagnetic positioning system), which induced signal is indicative of the position and/or orientation of the coil 182. The wire may be shielded, in an embodiment. The wire may terminate in two ends 178a, 178b that may be respectively electrically coupled with the distal contact pads 170a, 170b, in an embodiment. For example, in an embodiment, a first end 178a of the wire forming the coil 182 may be soldered to the first distal contact pad 170a, and a second end 178b of the wire forming the coil 182 may be soldered to the second distal contact pad 170b.

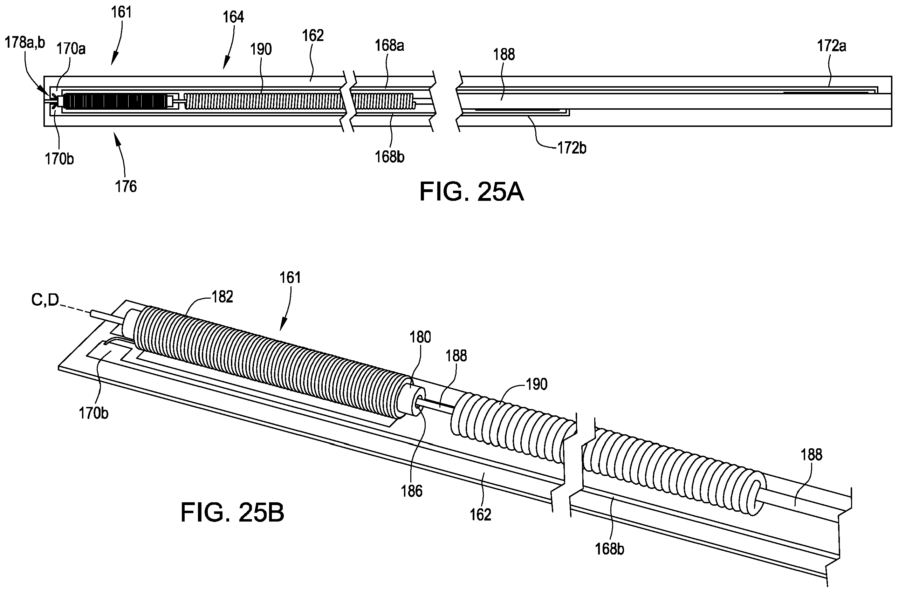

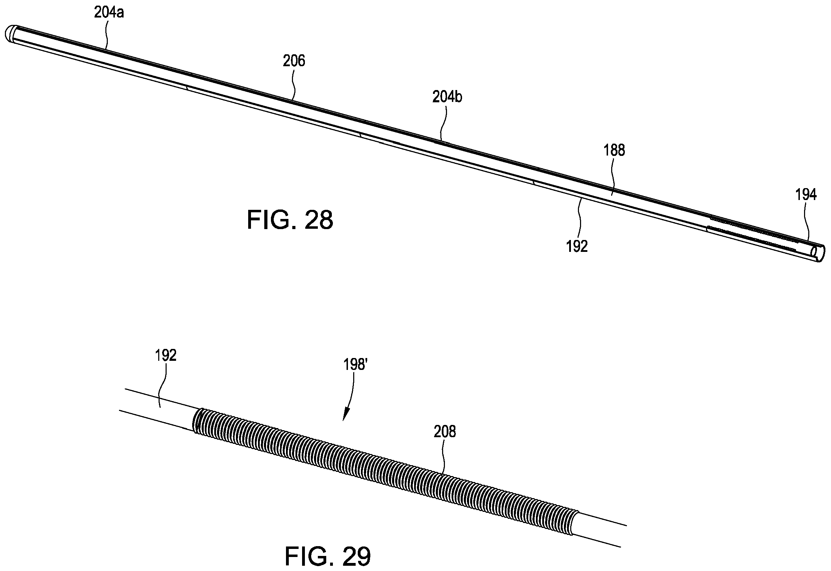

Figure 25A is a plan view of a further stage of build-up of the assembly 160, and Figure 25B is an isometric view of the distal end portion 161 of the assembly stage of Figure 28A. Referring to Figures 25A and 25B, a corewire 188 and a radiopaque coil 190 may be added to the assembly. The radiopaque coil 190 may be placed proximally of the sensor 176, in an embodiment.

The corewire 188 may be a cylindrical component that distributes bending stresses, tensile loads, and compressive loads over its length, reducing stress on the other components of the assembly, such as the traces 168a, 168b and the sensor 176. The corewire 188 may be or may include a metal, such as stainless steel, titanium, or nickel titanium alloys (*i.e.,* Nitinol). The corewire 188 may be a single continuous wire extending the entire axial length of the completed device, in an embodiment. Accordingly, the corewire may have a length of over 100 cm, in an embodiment. Further, the corewire may have a length of between 140 cm and 180 cm, in an embodiment. Still further, the corewire may have a length of between 145 cm and 155 cm or between 165 cm and 175 cm, in an embodiment. Alternatively, the corewire 188 may comprise a multi-piece construction, such as the construction described in United States patent application publication no. 2009/0192413. In either a single-piece or multi-piece embodiment, the corewire 188 may have a diameter that varies over its length. For example, the corewire 188 may be thicker at its proximal end than at its distal end, and may taper continuously or in stages. Alternatively, in an embodiment, the corewire 188 may have a constant diameter over its length.

The corewire 188 may define a longitudinal axis. The corewire 188 may be at the radial center of the assembly; accordingly, the longitudinal axis of the corewire 188 may also serve as the longitudinal axis D of the assembly 160 and of the completed device. In the figures, and for the remainder of this disclosure, for ease of reference, the longitudinal axis of the corewire 188 and the longitudinal axis of the assembly 160 and of the completed device are both referenced as axis D. It should be understood, however, that the corewire 188 may be disposed "off-center" in an embodiment of the assembly, such that the longitudinal axis of the corewire 188 is different from the longitudinal axis D of the completed device.

The radiopaque coil 190 may provide increased visibility of the device on x-rays as well as structural support. The radiopaque coil 190 may include a highly radiopaque material, in an embodiment, such as platinum.

The corewire 188 may be placed so as to extend through the radiopaque coil 190 and through the sensor 176, in an embodiment. For example, in an embodiment, the corewire 188 may be extended through the lumen 186 of the sensor core 180.

The corewire 188 may have the same length as the substrate 162, in an embodiment, and may extend so as to co-terminate at its distal and proximal ends with the substrate 162. Alternatively, the corewire 188 may be longer or shorter than the substrate 162, and may extend further proximally and/or distally, or the substrate 162 may extend farther proximally and/or distally.

Figure 26 is an isometric view of the distal end portion of a further stage of build-up of the assembly, with portions cut away for clarity of illustration. As shown in Figure 26, the substrate 162 may be rolled so as to form a tube 192, and an exterior layer 194 (which may be referred to herein as a jacket 194) may be placed radially about the substrate tube 192. An atraumatic tip 196 may be coupled with the distal tip of the corewire 188 and/or the substrate tube 192.

The substrate tube 192 may be formed from the entire length of the substrate 162, in an embodiment. Accordingly, the substrate tube 192 may span the entire axial length of the assembly. The substrate 162 tube may be radially symmetric around the longitudinal axis D of the corewire 188 (and, thus, about the corewire 188 itself), in an embodiment. The two ends of the substrate 162 that meet so as to form the tube may be coupled with each other, in an embodiment (*e.g.,* with adhesive), or they may remain "free" from each other, with the shape of the tube enforced by layers of the assembly applied on the radial exterior of the substrate tube, such as the jacket 194.

The jacket 194 may include a polymer or other material. For example, in an embodiment, the jacket 194 may be or may include a polyether block amide material, such as one commercially available under the trade name Pebax® from Arkema, Inc. The jacket 194 may be provided over substantially the entire length of the assembly 160, in an embodiment, and thus may cover the outer surface of the entirety of the substrate tube 192, in an embodiment. The jacket 194 may have a thickness and/or durometer (*i.e.,* stiffness) that varies over its length, in an embodiment. Alternatively, the thickness and/or durometer of the jacket 194 may be constant over its length. The jacket 194 may be applied on the assembly according to any appropriate technique. For example, in an embodiment, the jacket 194 may be extruded and then placed over the assembly 160 (*e.g.,* over the substrate tube 192). Alternatively, the jacket 194 maybe reflowed or otherwise melt-processed directly onto the assembly 160. The jacket 194 may be radially symmetric about the longitudinal axis D of the assembly 160, in an embodiment.

The tip 196 may provide an atraumatic interface between the patient's anatomy and the finished device. The atraumatic tip 196 may be formed from, for example, a polymer or an adhesive. The atraumatic tip 196 may be directly coupled (*e.g.,* with adhesive) to one or more of the corewire 188, the substrate tube 192, and the jacket 194, in an embodiment. For example, the atraumatic tip 196 may be integrally formed with the jacket 194, in an embodiment. Alternatively, the atraumatic tip 196 may be a molded plug that is bonded to an inner surface of the substrate tube 192 and/or an outer surface of the corewire 188, in an embodiment. Alternatively, the atraumatic tip may be formed by encapsulating the distal tip of the assembly 160 via dip coating in, for example, only, a polyurethane material such as Biothane® 228.

The distal end portion 161 of the assembly 160 may be generally radially symmetric about the longitudinal axis D, in an embodiment. That is, each of the corewire 188, the sensor 176, the substrate tube 192, the radiopaque coil 190, and the jacket 194 may comprise a hollow or solid cylindrical cross-section with the longitudinal axis D at its center. Such radial symmetry may advantageously provide relatively uniform bending characteristics in all directions and a consistent stress distribution for deflection in all directions.

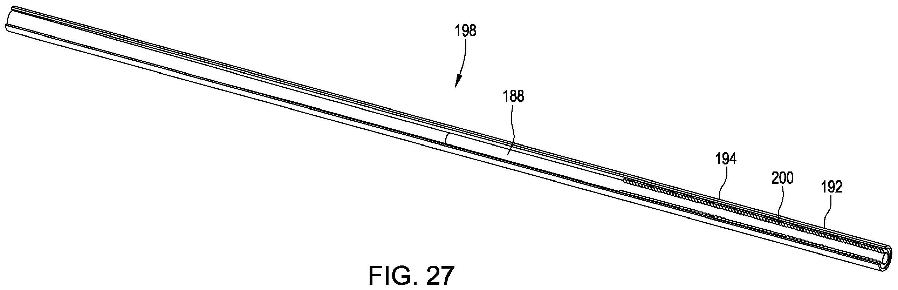

Figure 27 is an isometric view of an intermediate portion 198 of the assembly 160 at an equivalent stage of build-up as that shown in Figure 26. The intermediate portion 198 of the assembly 160 may include the corewire 188, a support coil 200 radially surrounding a portion of the corewire 188, and the substrate tube 192 radially surrounding the corewire 188 and support coil 200. The jacket 194 may further radially surround the corewire 188, support coil, and substrate tube.

The support coil 200 may be the same as the radiopaque coil 190 (*see* Figure 26), in an embodiment. That is, the radiopaque coil 190 may extend proximally to the intermediate portion 198 of the assembly 160 as serve as the support coil 200. Alternatively, the support coil 200 may be a separate coil from the radiopaque coil 190. In addition to, or as an alternative to, the support coil 200, another elongate (*e.g.,* tubular) support structure may be provided, such as a braided mesh of metal or polymer, for example.

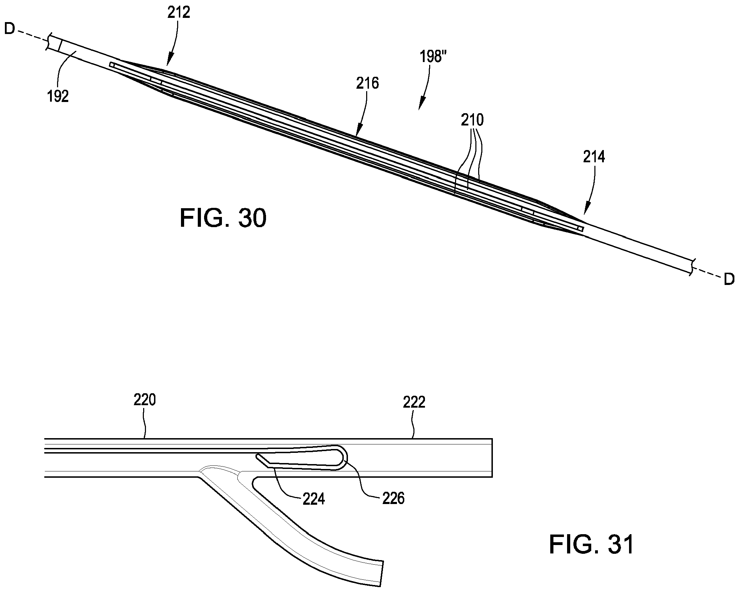

Figure 28 is an isometric view of the proximal end portion 202 of the assembly 160 at an equivalent stage of build-up as that shown in Figures 29 and 30. The proximal end portion 202 of the assembly 160 may include the corewire 188, the substrate tube 192, and the jacket 194. The proximal end portion 202 may also include one or more electrical contacts 204a, 204b, and an insulator 206 separating the one or more electrical contacts from each other.

The electrical contacts 204a, 204b may be electrically coupled with the traces 168a, 168b on the interior surface of the substrate tube 192. For example, the electrical contacts 204a, 204b may be electrically coupled with proximal contact pads 172a, 172b (*see* Figure 23A) at which the traces 168a, 168b terminate. In an embodiment, both the proximal contact pads 172a, 172b and the electrical contacts 204a, 204b may include electrically-conductive vias which may be electrically coupled with each other. Alternatively, the electrical contacts 204a, 204b may include terminals on an inner surface, which terminals may be electrically coupled with wires that are also electrically coupled with the proximal contact pads 172a, 172b. Alternatively, some other electrical coupling may be provided.

Figure 29 is an isometric view of an alternate embodiment of the intermediate portion 198' of the guidewire assembly. The intermediate portion embodiment 198' of Figure 29 may be the same as the intermediate portion 198 illustrated in Figure 27 except as otherwise described below.

The assembly intermediate portion 198' may include a baffle 208. The baffle 208 may include a plurality of concentric ridges having the longitudinal axis D of the device at their center. The baffle 208 may be provided in a longitudinal portion of the device for which a large amount of bending is expected, and may provide additional support for bending stress. As illustrated, the baffle may be disposed around the substrate tube 192, in an embodiment. Though not illustrated in Figure 29, the intermediate portion may further include the support coil 200 radially-inward of or radially-outward of the baffle 208, and/or the jacket 194 radially-outward of the baffle 208 (and, if provided, of the support coil).

Figure 30 is an isometric view of another alternate embodiment of the intermediate portion 198" of the assembly. The intermediate portion embodiment 198" of Figure 30 may be substantially the same as the intermediate portion 198 illustrated in and described with respect to Figure 27 except as otherwise described below.

The intermediate portion 198" may include one or more struts 210. Each strut 210 may be or may include a proximal end 212 coupled with the substrate tube 192, a distal end 214 coupled with the substrate tube 192, and a longitudinally-extending member 216 between the proximal and distal ends 212, 214. The longitudinal member 216 of each strut 210 may be substantially parallel with the longitudinal axis D of the device, in an embodiment. Though not illustrated in Figure 30, the intermediate portion 198" may further include the support coil radially-inward of or radially-outward of the struts 210, and/or the jacket 194 radially-outward of the struts 210 (and, if provided, of the support coil 200).

Figure 31 is a diagrammatic view of a guidewire 220 disposed within a vessel 222 of a patient, illustrating an exemplary use for the guidewire 220 that may be enabled by the techniques and assemblies of this disclosure. In an exemplary procedure, the guidewire 220 may be prolapsed within the vessel 222. That is, the distal end 224 of the guidewire 220 may be deflected 180°, as illustrated in Figure 31. The prolapse may be performed, for example, to place a sensor or therapeutic element on a medical device (*e.g.,* a catheter; not shown in Figure 31) that is placed over the guidewire as far distal as possible. For example, a pacing lead may be disposed a few millimeters proximal of the distal tip of a catheter; to place the pacing lead as far distal as possible during a procedure, the guidewire 220 may be prolapsed, and the catheter may be extended over the guidewire 220 so as to place the pacing lead at the apex 226 of the prolapse. The guidewire may be configured so that the apex 226 of the prolapse corresponds to a distal end portion 161 or intermediate portion 198, 198', 198" illustrated and/or described herein.

Traditional guidewire designs and assembly techniques may be inadequate to withstand the bending stress of a prolapse while maintaining electrical functionality for a sensor disposed at the distal end portion of the guidewire. In contrast, a guidewire according to the present disclosure may better distribute stresses from bending around a sensor, thus protecting the sensor itself from damage, and may also better maintain an electrical connection with the sensor through the use of traces 168a, 168b on a flexible substrate rather than a traditional twisted pair or other traditional wiring.

In one or more of the embodiments described above, it may be advantageous to provide increased anisotropic strength in a substrate. For example, it may be advantageous to provide increased strength along the longitudinal axis of the device.

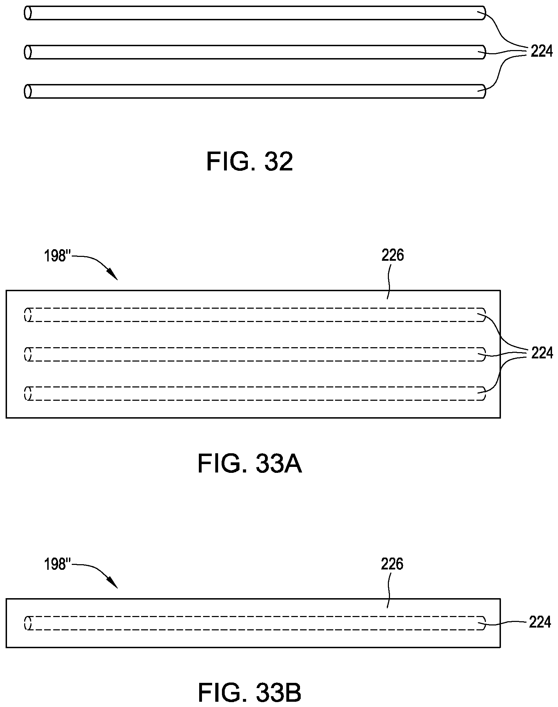

In an embodiment, a substrate may be strengthened by providing a plurality of nanowires in the substrate. Figures 32-33B illustrate an exemplary procedure for producing such a substrate 162'.

Figure 32 is a plan view of a plurality of nanowires 224 that may be laid substantially parallel to each other. Each nanowire 224 may comprise an elongate wire having a diameter on the order of tens of nanometers or less and may be made of a conductive, semiconductive, or insulating material. In an embodiment, one or more nanowires 224 may be conductive material and may be used to conduct electrical signals instead of or in addition to electrically-conductive traces on the substrate as described herein.

Each nanowire 224 may have a diameter of less than about twenty-five (25) micrometers, in an embodiment. In a further embodiment, each nanowire 224 may have a diameter that is less than a micrometer. In a further embodiment, each nanowire 224 may have a diameter of between one hundred (100) nanometers and one micrometer. It should be noted that the nanowires 224 are not shown to scale in the figures, and that a large number of nanowires 224 may be provided in a substrate 198".

The nanowires 224 may comprise one or more materials selected according to the desired diameter of the nanowires 224. For example, for nanowire diameters of less than about five hundred (500) nm, the nanowires 224 may comprise silver (such as, for example, ClearOhm® material commercially available from Cambrios Technologies Corporation), and/or copper. For such nanowire diameters, the center-to-center wire pitch may be between 500nm and 1000nm. In another example, nanowires 224 having diameters between five hundred (500) nm and one thousand (1000) nm may comprise copper and other materials produced using additive manufacturing processes. For such nanowire diameters, the center-to-center wire pitch may be between five hundred (500) nm and one thousand (1000) nm and may be manufactured according to additive wire manufacturing on a first substrate to form the wire which is then incorporated into the flexible substrate as set forth below. Alternatively, depending on process materials and the heat generated by the additive process, deposition, direct incorporation into the flexible substrate (*i.e.,* without first manufacturing on a first substrate) may be possible if the processing temperature does not exceed the material's melting point (or thermal degradation temperature). In another example, nanowires 224 having diameters of around twenty-five (25) micrometers or more, gold nanowires may be employed (*e.g.,* AW-14 gold bonding wire commercially available from Heraeus Materials Technology GmbH & Co. KG). For such nanowire diameters, center-to-center wire pitch may be about twenty-five (25) micrometers or more.

As shown in Figure 33A, which is a "top" view, and Figure 33B, which is a "side" view, a substrate body material 226 may be applied to the nanowires 224 so as to encapsulate the nanowires 224 in the finished substrate 198". The substrate body material 226 may be a polymer, in an embodiment, such as one or more of the polymers set forth previously in this disclosure for a substrate. The substrate body material 226 may be solution cast onto the nanowires 224, in an embodiment. For example, the substrate body material 226 may be solution cast according to the solution casting process commercially offered by Avalon Laboratories, LLC of Rancho Dominguez, California.

Although a number of embodiments have been described above with a certain degree of particularity, those skilled in the art could make numerous alterations to the disclosed embodiments without departing from the scope of this disclosure. For example, all joinder references (*e.g.,* attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting. Changes in detail or structure may be made without departing from the scope of the invention as defined in the appended claims.

## Claims

1. An elongate medical device (10) comprising:
an elongate body (12, 188) defining a longitudinal axis;
a sensor (182); and
a flexible tube (192) disposed about said longitudinal axis;
wherein said elongate body (188) is a corewire (188) that extends through said flexible tube (192),
wherein said sensor (182) comprises
a coil comprising a plurality of turns of wire, each turn of wire disposed about said axis, and
a sensor core (180), wherein said coil is wound on said core (180), said core defining a lumen (186) through which said corewire (188) extends,
**characterized in that** an electrically-conductive trace (34, 168a, 168b) is deposited on the flexible tube (192), wherein said coil is electrically coupled with said trace (168a, 168b).

2. The elongate medical device (10) of claim 1, wherein said flexible tube (192) comprises a longitudinal length that is greater than a longitudinal length of said sensor (182).

3. The elongate medical device (10) of claim 1 or 2, further comprising a radiopaque coil (190) disposed within the tube (192).

4. The elongate medical device (10) of any one of claims 1 to 3, further comprising an adhesive coupling said sensor (182) with said flexible tube (192).

5. The elongate medical device (10) of any one of claims 1 to 4, wherein said sensor (182) comprises a position sensor.

6. The elongate medical device (10) of any one of claims 1 to 5, wherein said elongate body (12) is a shaft.

7. The elongate medical device (10) of any one of claims 1 to 6, wherein said flexible tube (192) extends further in a proximal direction along said longitudinal axis than does said sensor (182).

8. The elongate medical device (10) of claim 7, wherein said flexible tube (192) has a length of at least one hundred centimeters.

9. The elongate medical device (10) of claim 4, further comprising an adhesive pad disposed on said flexible tube (192), said sensor (182) coupled with said adhesive pad.

## Patentansprüche

1. Langgestreckte medizinische Einrichtung (10), enthaltend:
einen langgestreckten Körper (12, 188), der eine Längsachse definiert;
einen Sensor (182); und
ein flexibles Rohr (192), das um die Längsachse herum angeordnet ist;
wobei der langgestreckte Körper (188) ein Kerndraht (188) ist, der sich durch das flexible Rohr (192) erstreckt,
wobei der Sensor (182) aufweist
eine Spule, die eine Vielzahl von Drahtwindungen aufweist, wobei jede Drahtwindung um die Achse herum angeordnet ist, und
einen Sensorkern (180), wobei die Spule auf den Kern (180) gewickelt ist, wobei der Kern ein Lumen (186) definiert, durch das sich der Kerndraht (188) erstreckt,
**dadurch gekennzeichnet, dass** eine elektrisch leitende Bahn (34, 168a, 168b) auf das flexible Rohr (192) aufgebracht ist, wobei die Spule mit der Bahn (168a, 168b) elektrisch gekoppelt ist.

2. Langgestreckte medizinische Einrichtung (10) nach Anspruch 1, wobei das flexible Rohr (192) eine Länge in Längsrichtung aufweist, die größer ist als eine Länge in Längsrichtung des Sensors (182).

3. Langgestreckte medizinische Einrichtung (10) nach Anspruch 1 oder 2, ferner enthaltend eine röntgenstrahlenundurchlässige Spule (190), die innerhalb des Rohrs (192) angeordnet ist.

4. Langgestreckte medizinische Einrichtung (10) nach einem der Ansprüche 1 bis 3, ferner enthaltend einen Klebstoff, der den Sensor (182) mit dem flexiblen Rohr (192) koppelt.

5. Langgestreckte medizinische Einrichtung (10) nach einem der Ansprüche 1 bis 4, wobei der Sensor (182) einen Positionssensor aufweist.

6. Langgestreckte medizinische Einrichtung (10) nach einem der Ansprüche 1 bis 5, wobei der langgestreckte Körper (12) ein Schaft ist.

7. Langgestreckte medizinische Einrichtung (10) nach einem der Ansprüche 1 bis 6, wobei sich das flexible Rohr (192) weiter in eine nahe Richtung entlang der Längsachse erstreckt als es der Sensor (182) tut.

8. Langgestreckte medizinische Einrichtung (10) nach Anspruch 7, wobei das flexible Rohr (192) eine Länge von wenigstens einhundert Zentimetern aufweist.

9. Langgestreckte medizinische Einrichtung (10) nach Anspruch 4, ferner enthaltend ein Klebepad, das auf dem flexiblen Rohr (192) angeordnet ist, wobei der Sensor (182) mit dem Klebepad gekoppelt ist.

## Revendications

1. Dispositif médical allongé (10) comprenant:
un corps allongé (12, 188) définissant un axe longitudinal ;
un capteur (182) ; et
un tube flexible (192) disposé autour dudit axe longitudinale ;
dans lequel ledit corps allongé (188) est un fil central (188) qui s'étend à travers ledit tube flexible (192),
dans lequel ledit capteur (182) comprend :
une bobine comprenant une pluralité de tours de fil, chaque tour de fil disposé autour dudit axe, et,
un noyau de capteur (180), dans lequel ladite bobine est enroulée sur ledit noyau (180), ledit noyau définissant une lumière (186) à travers laquelle ledit fil central (188) s'étend,
**caractérisé par le fait qu'**une trace électriquement conductrice (34, 168a, 168b) est disposée sur ledit tube flexible (192), ladite bobine est couplée électriquement à ladite trace (168a, 168b).

2. Dispositif médical allongé (10) selon la revendication 1, dans lequel ledit tube flexible (192) comprend une longueur longitudinale qui est plus grande qu'une longueur longitudinale dudit capteur (182).

3. Dispositif médical allongé (10) selon la revendication 1 ou 2, comprenant en outre une bobine radio-opaque (190) disposée à l'intérieur du tube (192).

4. Dispositif médical allongé (10) selon l'une quelconque des revendications 1 à 3, comprenant en outre un adhésif couplé audit capteur (182) avec ledit tube flexible (192).

5. Dispositif médical allongé (10) selon l'une quelconque des revendications 1 à 4, dans lequel ledit capteur (182) comprend un capteur de position.

6. Dispositif médical allongé (10) selon l'une quelconque des revendications 1 à 5, dans lequel ledit corps allongé (12) est une tige.

7. Dispositif médical allongé (10) selon l'une quelconque des revendications 1 à 6, dans lequel ledit tube flexible (192) s'étend davantage dans une direction proximale le long dudit axe longitudinal que ne le fait ledit capteur (182).

8. Dispositif médical allongé (10) selon la revendication 7, dans lequel ledit tube flexible (192) a une longueur d'au moins cent centimètres.

9. Dispositif médical allongé (10) selon la revendication 4, comprenant en outre un tampon adhésif disposé sur ledit tube flexible (192), ledit capteur (182) étant couplé audit tampon adhésif.
